# EUROPEAN PATENT SPECIFICATION

(11) **EP 2 766 045 B1**
(45) Date of publication and mention of the grant of the patent: **07.12.2016**
(21) Application number: 12840745.9
(22) Date of filing: 15.10.2012
(51) Int. Cl.: C07K 16/30, A61K 39/395, A61K 38/10, A61K 38/16, A61K 48/00, A61P 35/00, A61K 39/00, A61K 45/06, A61K 47/48

(54) **TREATMENT OF BREAST CANCER WITH COMPANION DIAGNOSTIC**
BEHANDLUNG VON BRUSTKREBS MIT BEGLEITENDER DIAGNOSE
TRAITEMENT PERSONNALISÉ DU CANCER DU SEIN PAR UN DIAGNOSTIC

(30) Priority: 13.10.2011 US 201161546982 P
(43) Date of publication of application: 20.08.2014
(73) Proprietor: The Regents of the University of California, Oakland, CA 94607-5200 (US)
(72) Inventor: WADEHRA, Madhuri, Manhattan Beach, CA 90266 (US); BRAUN, Jonathan, Tarzana, CA 91356 (US)
(74) Representative: O'Neill, Michelle
(86) International application number: PCT/US2012/060288
(87) International publication number: WO 2013/056248

(56) References cited:
- WO-A2-2009/048980
- WO-A2-2011/112953
- M. FU ET AL.: "Epithelial membrane protein-2 is a novel therapeutic target in ovarian cancer.", CLINICAL CANCER RESEARCH, vol. 16, no. 15, 1 August 2010 (2010-08-01), pages 3954-3963, XP002738317, USA
- K. SHIMAZAKI ET AL.: "Diabodies targeting epithelial membrane protein 2 reduce tumorigenicity of human endometrial cancer cell lines.", CLINICAL CANCER RESEARCH, vol. 14, no. 22, 15 November 2008 (2008-11-15), pages 7367-7377, XP002738318, USA

## Description

### FIELD OF THE INVENTION

This invention relates to anti-EMP2 antibodies, their pharmaceutical compositions and methods for using them in the detection and treatment of cancers that overexpress EMP2, such as triple negative breast cancers.

### BACKGROUND OF THE INVENTION

Breast cancer remains the most common malignancy among women worldwide. Breast cancer is a heterogeneous disease, which exhibits a wide range of clinical behaviors, prognoses, and histologies (Tavassoli F, Devilee P, editors. (2003) WHO Classification of Tumors. Pathology & Genetics: Tumors of the breast and female genital organs. Lyon (France): IARC Pres). Breast cancer is the abnormal growth of cells that line the breast tissue ducts and lobules and is classified by whether the cancer started in the ducts or the lobules and whether the cells have invaded (grown or spread) through the duct or lobule, and by the way the cells look under the microscope (tissue histology). It is not unusual for a single breast tumor to have a mixture of invasive and in situ cancer.

Molecular classification of breast cancer has identified specific subtypes, often called "intrinsic" subtypes, with clinical and biological implications, including an intrinsic luminal subtype, an intrinsic HER2-enriched subtype (also referred to as the HER2⁺ or ER⁻/HER2⁺ subtype) and an intrinsic basal-like breast cancer (BLBC) subtype. (Perou et al. 2000). Identification of the intrinsic subtypes has typically been accomplished by a combination of methods, including (1) histopathological detection, (2) estrogen receptor (ER), progesterone receptor (PR) and human epidermal growth factor receptor 2 (HER2) expression status and (3) detection of characteristic cellular markers.

Basal-like breast cancer (BLBC), which expresses genes characteristic of basal epithelial cells in the normal mammary gland, comprises up to 15%-25% of all breast cancers (Kreike et al. 2007) and is associated with the worst prognosis of all breast cancer types. BLBCs underexpress estrogen receptor (ER⁻), progesterone receptor (PR⁻), and human epidermal growth factor receptor 2 (HER2⁻) and encompass 60% to 90% of so-called "triple negative" (ER⁻/PR⁻/HER2⁻) breast cancers. Although most basal-like breast cancers are often referred to as triple negative based on the expression status of ER, PR and HER2, not all basal-like breast cancers are triple negative.

Thus, the intrinsic basal-like breast cancer subtype may be further subdivided into at least three distinct subtypes described herein as "hybrid" basal-like breast cancer subtypes. In addition to a hybrid triple negative subtype, the hybrid basal-like breast cancer subtypes have profiles that resemble both basal-like breast cancer and at least one other breast cancer molecular subtype. For example, hybrid basal-like subtypes can include a hybrid basal-like/HER2⁺ subtype that has a receptor profile of ER⁻/PR⁻/HER⁺, a hybrid basal-like/luminal subtype that has a receptor profile of ER⁺/PR^{-or+}/HER^{-or+}, and a hybrid basal-like/triple negative subtype that has a receptor profile of ER⁻/PR⁻/HER⁻.

The intrinsic luminal breast cancer subtype is characterized by expression or overexpression of ER and/or PR (ER⁺ and/or PR⁺). The luminal subtype can be further subdivided based on HER2 status into the luminal A subtype, which is additionally characterized by underexpression of HER2 (ER⁺/PR^{+or-}/HER⁻), and luminal B subtype, which is additionally characterized by overexpression of HER2 (ER⁺/PR^{+or-}/HER⁺). Intrinsic luminal subtypes are often considered to be the most treatable breast cancer subtype and are associated with the best prognosis.

Whereas ER and HER2 guide treatment of luminal and HER2 breast cancers, respectively, chemotherapy remains the only modality of systemic therapy for BLBC. Preferentially affecting younger women, particularly African American women, BLBCs are associated with high histologic grade, aggressive clinical behavior, and a high rate of metastasis to the brain and lung (Carey et al. 2006). Unlike other breast cancer subtypes, there seems to be no correlation between tumor size and lymph node metastasis in BLBCs (Dent et al. 2007).

BLBCs are associated with expression of basal cytokeratins (CK5/6, CK14, and CK17), epidermal growth factor receptor (EGFR), c-kit, and p53 and associated with the absence of ER, PR, and HER2 expression. With a large variety of associated genes, BLBCs have been defined differently in different studies using a set of diagnostic markers. For example, Nielsen et al. defined BLBC on the basis of negative ER and negative HER2 expression in addition to positive basal cytokeratin, EGFR, and/or c-kit expression (Nielsen et al. 2004). On the other hand, other groups have defined BLBC on the basis of on a combination of negative ER, and negative HER2 expression and positive CK5, P-cadherin, and p63 expression (Elsheikh et al. 2008) or positive vimentin, EGFR, and CK5/6 expression (Livasy et al. 2006). These different technical approaches in combination with widely varying patient cohorts may explain the inconsistent experimental results for these markers.

Identification of the basal-like subtype using immunohistochemistry (IHC) for detecting hormone receptors alone is less desirable than detecting a theranostic biomarker, because identification is based on the absence of IHC staining for estrogen receptor (ER), progesterone receptor (PR), and human epidermal growth factor receptor 2 (HER2) rather than the presence of a specific tumor marker or markers. Its diagnosis is more one of exclusion rather than inclusion.

Important molecular characterizations have been made over the last decade which assist in prediction and treatment of breast cancer. These include BRCA1 mutation status as well as the expression of the estrogen receptor (ER) and epidermal growth factor type 2 receptor (ERBB2 or Her-2/neu). Gene expression profiling has recently identified specific breast cancer subgroups, each with a distinct molecular signature. This molecular classification system divides breast cancers into four biologically different subtypes: 1) normal/non-cancerous; 2) estrogen-receptor (ER)-positive luminal breast cancer; 3) ER-negative, HER2/neu overexpressing breast cancer; and 4) ER-negative breast cancer, with expression of basal-type cytokeratins (CK 5/6, 14, 17), p53, and overexpression of epidermal growth factor receptor (EGFR).

Treatment with Her-2/neu and ER modulators have shown clinical promise for tumors expressing these receptors. However, only approximately 15% of individuals have tumors with Her-2/neu over-expression thereby limiting the utility of this treatment in the general population. Furthermore, while 70% of breast cancers express ER, loss of expression and/or resistance to anti-ER therapies is a major issue.

A particularly aggressive type of breast cancer lacks expression of ER, Her-2/neu and the progesterone receptor (PR). This aggressive type of breast cancer is often referred to as triple negative breast cancer (TNBC). While this variant occurs in a minority of patients (10-15%), it accounts for approximately half of all breast cancer deaths.

Triple negative breast cancers are typically observed in young African American women and Caucasian women who carry a mutation in the BRCA1 gene. Triple negative breast cancers are typically ER/PR(-/-), Her2/neu(-/-), EGFR(+), p53(-) and cytokeratin 5/6 (+I+).

WO2011/112953 discloses an ErbB3 inhibitor for use in the treatment of triple negative breast cancer. While some triple negative breast cancers respond to chemotherapy, a subset of triple negative breast cancers are chemotherapy-resistant and highly metastatic, carrying with it an extremely poor prognosis. Therefore, identifying new molecular targets expressed in this aggressive subtype is of high priority remains a need in the art.

As reported herein, the epithelial membrane protein-2 (EMP2) is overexpressed in triple negative breast cancers. EMP2 is a tetraspan protein belonging to the growth arrest specific-3 (GAS3) family. Functionally, EMP2 associates with and modulates the localization and activity of both integrin αvβ3 and focal adhesion kinase (FAK). EMP2 (SEQ ID NO:1) is expressed at high levels in epithelial cells of the lung, eye, and genitourinary tracts. Like several tetraspan proteins (CD9, CD81, PMP22), EMP2 in murine fibroblasts is localized to lipid raft domains. EMP2 controls cell surface trafficking and function of certain integrins, GPI-linked proteins, and class I MHC molecules, and reciprocally regulates caveolin expression. (see, Claas et al., J Biol Chem 276:7974-84 (2001); Hasse et al., J Neurosci Res 69:227-32 (2002); Wadehra et al., Exp Mol Pathol 74:106-12 (2003); Wadehra et al., Mol Biol Cell 15:2073-2083 (2004); Wadehra et al., J Biol Chem 277:41094-41100 (2002); and Wadehra et al., Clin Immunol 107:129-136 (2003)).

SEQ ID NO:1 (ACCESSION P54851) MLVLLAFIIA FHITSAALLF IATVDNAWWV GDEFFADVWR ICTNNTNCTV INDSFQEYST LQAVQATMIL STILCCIAFF IFVLQLFRLK QGERFVLTSI IQLMSCLCVM IAASIYTDRR EDIHDKNAKF YPVTREGSYG YSYILAWVAF ACTFISGMMY LILRKRK

EMP2 appears to regulate trafficking of various proteins and glycolipids by facilitating transfer of molecules from post-Golgi endosomal compartments to appropriate plasma membrane locations. Specifically, EMP2 is thought to facilitate the appropriate trafficking of select molecules into glycolipids-enriched lipid raft microdomains (GEMs) (Wadehra et al., Mol Biol Cell 15:2073-83 (2004)). GEMs are cholesterol rich microdomains which are often associated with chaperones, receptosomes, and protein complexes that are important for efficient signal transduction (Leitinger et al., J Cell Sci 115:963-72 (2002); Moffett et al., J Biol Chem 275:2191-8 (2000)). Moreover, GEMs are involved in correct sorting of proteins from the Golgi apparatus to plasma membrane (Abrami et al., J Biol Chem 276:30729-36 (2001); Galbiati et al., Cell 106:403-11 (2001); Gruenberg et al., Curr Opin Cell Biol 7: 552-63 (1995)). In this respect, modulation of EMP2 expression levels or its location on the plasma membrane alters the surface repertoire of several classes of molecules including integrins, focal adhesion kinase, class I major histocompatibility molecules and other immunoglobulin super-family members such as CD54 and GPI-linked proteins (Wadehra et al., Dev Biol 287:336-45 (2005); Wadehra et al., Clinical Immunology 107:129-36 (2003); Morales et al., Invest Opthalmol Vis Sci (2008)).

EMP2 expression is associated with EMP2 neoplasia (Wadehra et al., Cancer 107:90-8 (2006)). In endometrial cancer, for example, EMP2 is an independent prognostic indicator for tumors with poor clinical outcome. EMP2 positive tumors, compared to EMP2 negative tumors, had a significantly greater myometrial invasiveness, higher clinical state, recurrent or persistent disease following surgical excision, and earlier mortality. WO2009/048980 discloses human antibodies and diabody versions thereof against EMP2 useful for treating chlamydia infection and endometrial cancer. Human anti-EMP2 diabodies, KS49 and KS83 for use in the treatment of ovarian cancer are described in Fu et al., Clinical Cancer Research, vol. 16, no.15, pages 3954-3963, 2010. Human anti-EMP2 diabodies which reduce the tumorigenicity of human endometrial cancer are described in Shimazaki et al., Clinical Cancer Research, Vol. 14, No. 22, pages 7367-7377, 2008.

Based on studies described herein it is now shown that EMP2 can be used as a target in the treatment of triple negative breast cancer. Accordingly, EMP2 polypeptides, anti-EMP2 antibodies, and EMP2 siRNA can be used to diagnose and treat triple negative breast cancers. As discussed above, there remains a large need for methods and compositions which are useful in the prevention, treatment, and modulation of triple negative breast cancers. Accordingly, this invention provides novel compositions and methods for meeting these and other needs.

### BRIEF SUMMARY OF THE INVENTION

The invention relates to an antibody that specifically binds to an epitope in the second extracellular loop of EMP2 wherein the epitope comprises the amino acid sequence DIHDKNAKFYPVTREGSYG for use in the treatment of breast cancer wherein the breast cancer comprises a triple negative breast cancer tumor.

In a specific embodiment, the the antibody further comprises a physiological acceptable carrier or a pharmaceutically acceptable carrier

In one embodiment, the anti-EMP2 antibody competes with an antibody comprising the heavy and light chain variable regions of a KS49, a KS41, a KS83, or a KS89 diabody.

In one embodiemnt, the anti-EMP2 antibody shares 90% amino acid identity with heavy and light chain variable regions of a KS49, a KS41, a KS83, or a KS89 diabody.

In one embodiment, the anti-EMP2 antibody comprises CDR sequences identical to those of a KS49, a KS41, a KS83, or a KS89 diabody.

The invention also relates to a composition comprising an antibody according to the invention and i) at least one additional anti-cancer agent; and/or ii) a companion diagnostic for use in the treatment of breast cancer wherein the breast cancer comprises a triple negative breast cancer tumor

In a specific embodiment, at least one additional anti-cancer agent is selected from the group consisting of platinum-based chemotherapy drugs, taxanes, tyrosine kinase inhibitors, anti-EGFR antibodies, anti-ErbB2 antibodies, and combinations thereof.

In a specific embodiment, at least one additional anti-cancer agent is an EGFR inhibitor. In a specific embodiment, the EGFR inhibitor is an anti-EGFR antibody. In a specific embodiment, the anti-EGFR antibody is cetuximab. In a specific embodiment, the anti-EGFR antibody is selected from the group consisting of matuzumab, panitumumab, and nimotuzumab.

In one embodiment, the EGFR inhibitor is a small molecule inhibitor of EGFR signaling. In a specific embodiment, the small molecule inhibitor of EGFR signaling is selected from the group consisting of gefitinib, lapatinib, canertinib, pelitinib, erlotinib HCL, PKI-166, PD158780, and AG 1478.

In one embodiment, at least one additional anti-cancer agent is a VEGF inhibitor.

In a specific embodiment, the VEGF inhibitor comprises an anti-VEGF antibody. In a specific embodiment, the anti-VEGF antibody is bevacizumab

In any of the embodiments, the tissue, cancer, subject, or patient to be treated is human or mammalian. In one embodiment, the treatment is of a patient which is a human. In one embodiment, the companion diagnostic wherein the companion diagnostic comprises an anti-EMP2 antibody.

In certain embodiments, the EMP2 antibodies may be used in diagnosis, prognosis, or the treatment of a cancer alone or when conjugated with an effector moiety. EMP2 antibodies conjugated with toxic agents, such as ricin, as well as unconjugated antibodies, may be useful therapeutic agents naturally targeted to EMP2 bearing cancer cells. Such antibodies can be useful in blocking invasiveness of the cancer or in vaccine therapies for the cancer.

### BRIEF DESCRIPTION OF THE DRAWINGS

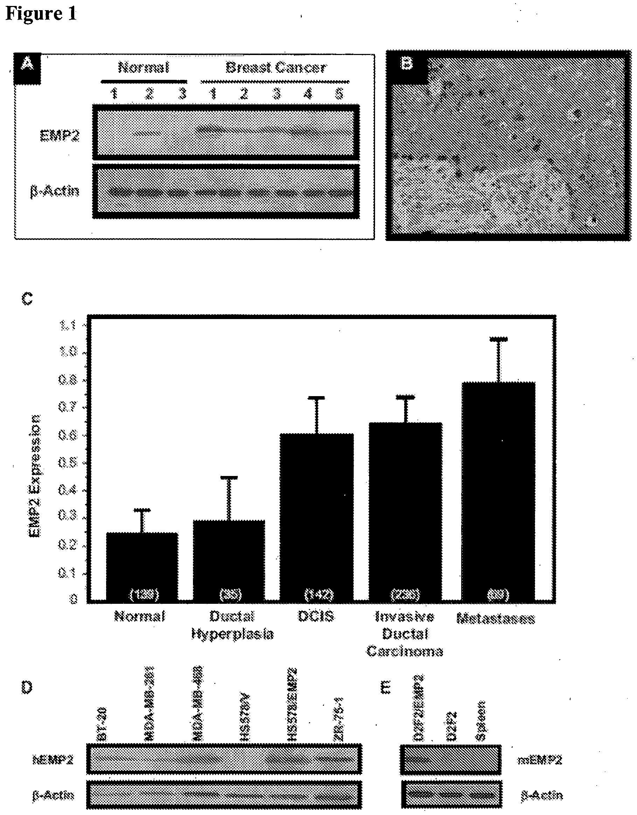

**Figure 1** depicts EMP2 expression is stratified by histologic type and stage. (A) Western blot analysis was performed on whole tissue homogenates from normal and tumor regions of the breast. EMP2 (18kD) is highly expressed in the breast tumor. Weak expression was visualized in one normal patient. (B) Normal and breast tumor tissue were stained for EMP2 expression using a tissue array. A representative patient with high EMP2 staining within a tumor is shown. (C) The mean integrated intensity of EMP2 protein expression for each category is shown using bar plots. The error bars represent the standard error of the mean; n is number of sample. EMP2 expression was significantly increased in ductal carcinoma in situ (DCIS, P < 0.01), invasive ductal carcinoma (IDC, P < 0.01), and metastasis (P < 0.01) compared to normal. Numbers represent the number of spots analyzed. (D) Expression of EMP2 in a panel of breast cancer cells was evaluated by Western blot analysis. HS578/EMP2 cells were generated to stably overexpress EMP2. (E) Expression of EMP2 was evaluated in the murine mammary cell D2F2, cells which overxpress EMP2 (D2F2/EMP2), as well in a representative murine spleen homogenate.

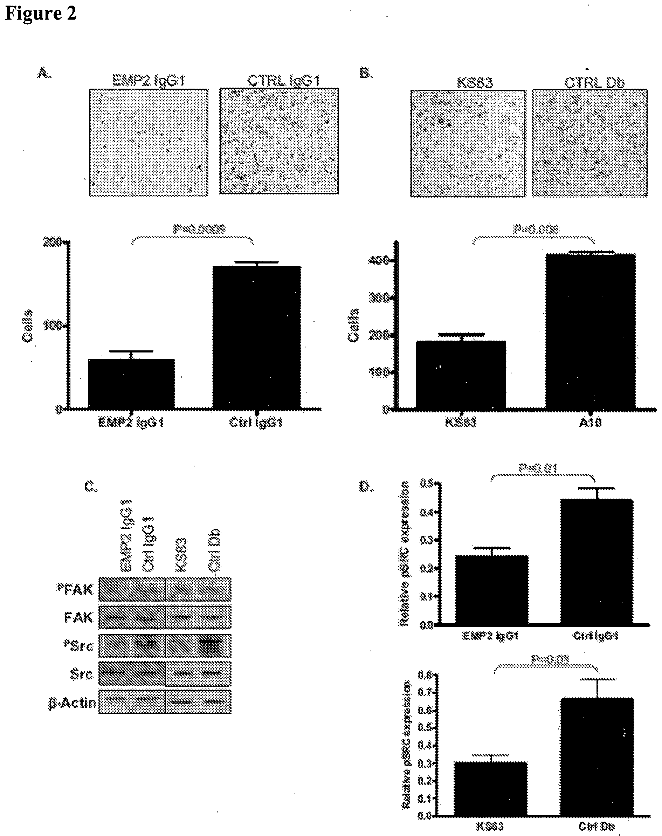

**Figure 2** depicts reduced cellular invasion in the presence of anti-EMP2 IgG1 antibodies and diabodies. (A) 60 µg/ml anti-EMP2 IgG1 or control IgG or (B) 20 µg/ml EMP2 diabody KS83 or control diabody A10 were added to cells for 1 hr. Invasion was measured as the number of cells that migrated through the transwell. Top panel, Cells were visualized using crystal violet. Bottom panel, Averaged results from 3 experiments. (C) 60 µg/ml anti-EMP2 IgG1 or control IgG was added to MDA-MB-468 cells for 1 hr. Alternatively, 20 ug/ml EMP2 KS83 or control diabody were added. Cells were then plated for 12 hrs and lysed. Proteins were separated by SDS-PAGE and probed using the indicated antibodies. The experiment was repeated three times, and representative image is shown. (D) pSRC expression from three experiments were quantified using Image J, and the data was normalized relative to total Src levels. Treatment with anti-EMP2 IgG1 or KS83 significantly reduced pSRC expression. Values are averages (±SEM).

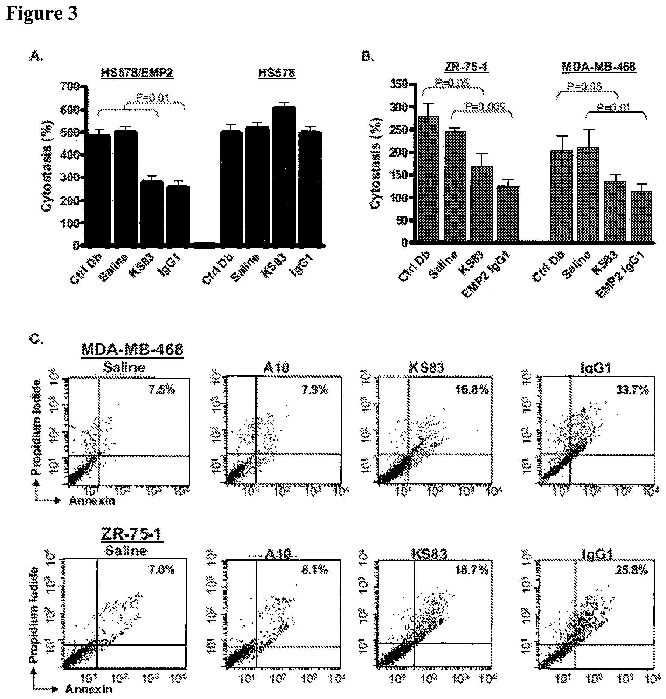

**Figure 3** depicts the induction of cytostasis in the presence of Anti-EMP2 IgG1 antibodies and diabodies. (A) HS578 cells do not express any endogenous EMP2 expression, and HS578/EMP2 cells were engineered to overexpress EMP2. 60 µg/ml anti-EMP2 IgG1 or control IgG; 20 µg/ml EMP2 diabody KS83 or control diabody were added to the above cells for 72 hours. Cellular viability was determined using trypan blue exclusion, and values represent results from 3 independent experiments (±SEM). (B) ZR-75-1 and MBA-MD-468 cells were treated as above to determine cellular viability. Values are averages (±SEM, n=3). (C) EMP2 diabodies and IgG1 promote apoptosis. ZR-75-1 (top panels) and MBA-MD-468 (bottom panels) cells were incubated with 20 µg/mL KS83, 20 µg/mL control diabody, 60 µg/ml anti-EMP2 IgG1, or 60 µg/mL control IgG. Cells were washed and stained with Annexin V and propidium iodide. Staining is expressed as the % Annexin V-7-aminoactinomycin D-positive cells above the isotype control. The experiment was repeated three times with similar results. A representative graph is shown.

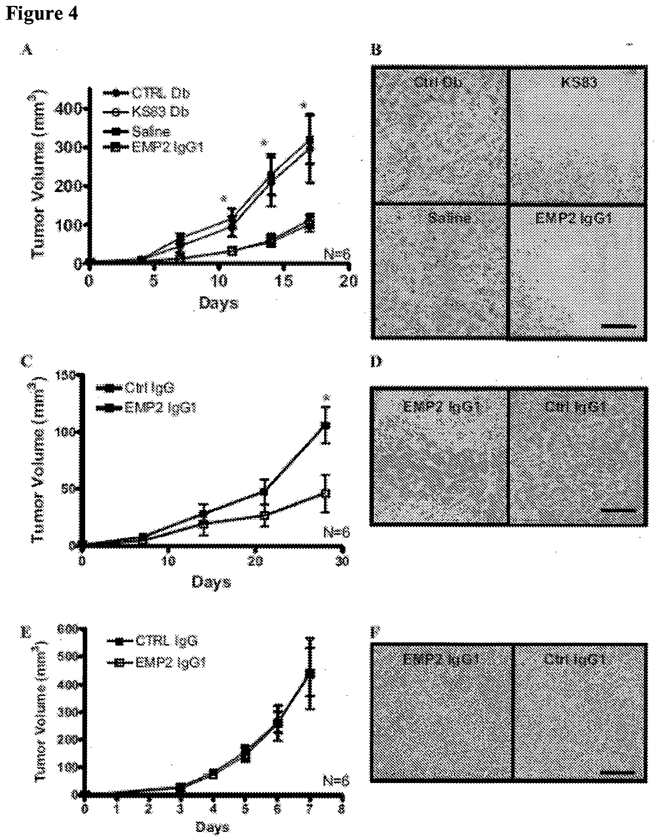

**Figure 4** depicts that the targeting EMP2 reduces tumor load. (A) MDA-MB-468 cells were injected s.c. into nude BALB/c female mice. When tumors were detectable, they were injected i.t. with molar equivalent amounts of diabody (1 mg/kg), IgG1 antibody (3 mg/kg), or the indicated controls (Day 0). Mice were injected twice a week, and tumor volume was monitored using calipers. n=6. *, p<0.05 for both KS83 and anti-EMP2 IgG1 treatment compared with diabody A10 and sterile saline, respectively. (B) At day 18, tumors were excised, formalin fixed, and paraffin embedded. Tumor histology was assessed by hemotoxylin and eosin staining. A representative image is shown. Magnification: 20X. Scale bar = 100 µm. (C) MDA-MB-468 cells were injected as above into nude BALB/c mice as above. Mice were systemically treated with 5mg/kg anti-EMP2 IgG1 or control IgG every week. Tumor volume was determined using calipers, and values represent averages (±SEM, n = 6). Comparison by Student's t-test, * p<0.05. (D) At day 18, tumors were excised, formalin fixed, and paraffin embedded. Tumor histology was assessed by hemotoxylin and eosin staining. A representative image is shown. Magnification: 20X. Scale bar = 100 µm. (E) Ramos cells were injected into the s.c. into nude BALB/c mice. As tumors grow rapidly, they were treated systemically twice in the week (Day 0 and 3) with 5mg/kg EMP2 IgG1 or control IgG. Tumor volume values are averages (±SEM, n = 6). (F) Representative hematoxylin and eosin staining at day 28. Magnification: 20X. Scale bar = 100 µm.

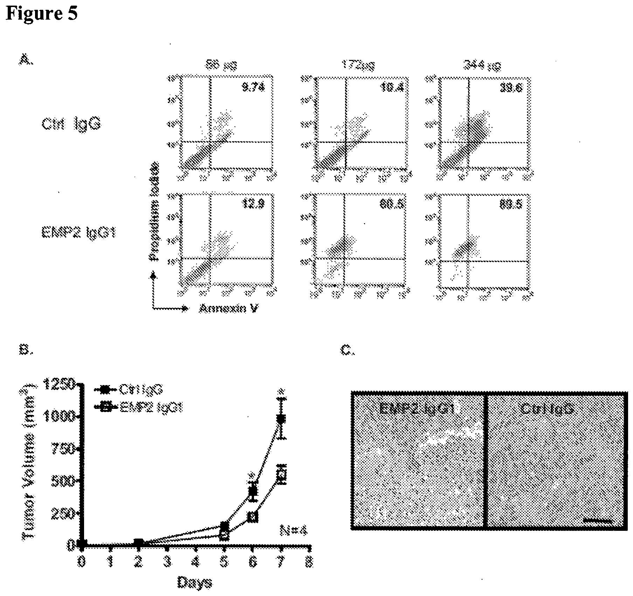

**Figure 5** depicts the reduction of tumor load in orthotopic mammary tumors in the presence of anti-EMP2 antibodies. (A) D2F2 cells are susceptible to apoptosis when treated with anti-EMP2 IgG1 compared to the control IgG. Cells were stained with annexin-V and propidium iodide, and then analyzed by flow cytometry. Images are representative of three independent experiments which showed similar results. (B) D2F2 cells were injected into the mammary pad of BALB/c mice. When tumors were first detectable, mice were systemically injected with 10 mg/kg anti-EMP2 or control IgG twice in the week (Day 0 and 3). Tumor volume was calculated using calipers N = 6. *, p<0.05, comparison by Student's t test. (C) Representative images of Day 7 tumors stained with hemotoxlin and eosin. Magnification: 20X. Scale bar, 100µm.

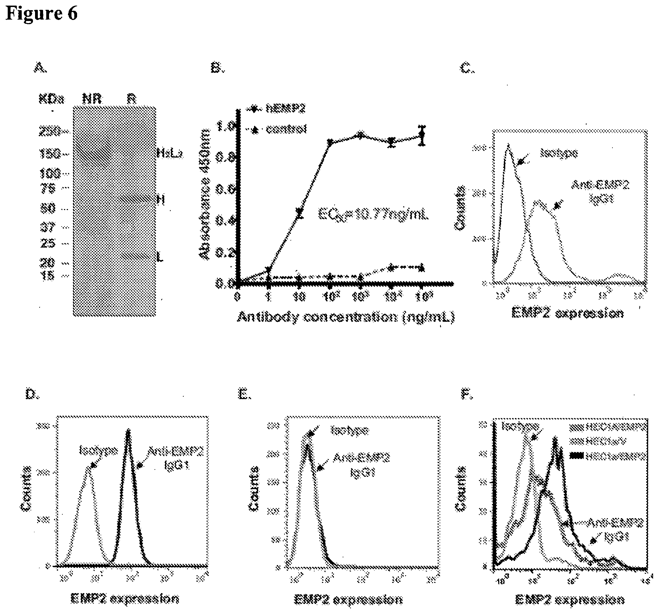

**Figure 6** depicts the characterization of a recombinant anti-EMP2 IgG1 antibody. An anti-EMP2 IgG1 humanized antibody was constructed. To verify our construct, the anti-EMP2 IgG1 antibody was sequenced and analyzed by SDS-PAGE under non-reducing (NR) and reducing (R) conditions. The 150 KDa band appeared, which exhibited a molecular weight corresponding to our anti-EMP2 IgG1. Anti-EMP2 IgG1 migrated as monomers under reducing condition at an expected 66 kDa size, corresponding to the H-chain (H) and 20 kDa L-chain (L) bands. To characterize the specificity of the anti-EMP2 IgG1, the binding of the full length anti-EMP2 antibody was measured against EMP2 peptide as well as to native protein. Using a human EMP2 peptide, serial dilutions of the native antibody revealed an EC₅₀ of 10.8 ng/ml (B). To further determine the binding characteristics of the anti-EMP2 IgG1, its binding to native EMP2 was determined using flow cytometry. Anti-EMP2 IgG1 recognized both murine EMP2 present on D2F2 cells (C) and human EMP2 on an endometrial carcinoma cell line which overexpresses EMP2, HEC-1a/EMP2 cells (D). The binding of the antibody was specific as it did not bind the EMP2 negative cell line Ramos (E). Finally, the sensitivity of the antibody was measured using HEC-1A wild type and HEC-1A/EMP2 cells which express a two to four-fold increase in total EMP2 levels. Anti-EMP2 IgG1 detected a difference in surface expression between the two cell lines (F).

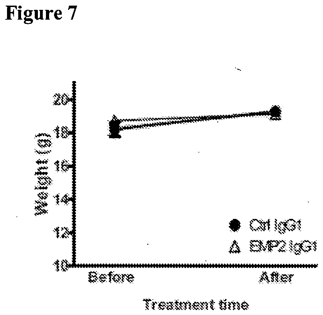

**Figure 7** depicts the lack of systemic toxicity exhibited by antibodies to EMP2. Anti-EMP2 diabodies (55 kDa) do not exhibit systemic toxicity. The full-length antibody differs from diabody in its valency, molecular size, and presence of immunoglobulin constant region domains which serve to modify the in vivo properties such as tumor uptake and interaction with immune system. To determine if the anti-EMP2 IgG1 produces any histological or sera toxicity in normal BALB/c mice, mice were injected weekly with 10 mg/kg anti-EMP2 IgG1 or sterile saline for seven weeks. Animals treated with either the control of anti-EMP2 IgG1 exhibited no significant differences in weight. Although a slight change in AST and cholesterol was observed, all changes were within the normal range found in mice as provided in Table 1 below. N=4.

**Table 1**

| | | **Treatment** | |
|---|---|---|---|
| **Serum Protein** | **Normal Mouse Reference** | **Control IgG** | **EMP2 IgG1** |
| **Albumin (g/dL)** | **2.5-5.4** | 3.1 ± 0.2 | 3.0 ± 0.2 |
| **Alkaline Phosphatase (U/L)** | **13-291** | 55 ± 9.2 | 73.5 ± 6.4 |
| **ALT (U/L)** | **7-227** | 39.7 ± 3.1 | 49.5 ± 0.7 |
| **AST (U/L)** | **34-329** | 112.3 ± 20 | 148.0 ± 28.3 |
| **Total Bilirubin (mg/dL)** | **0.1-1.1** | 0.3 ± 0.1 | 0.3 ± 0.2 |
| **Direct Bilirubin (mg/dL)** | **-** | 0.3 ± 0.1 | 0.2 ± 0.1 |
| **BUN (mg/dL)** | **2.0-5.4** | 16.0 ± 3 | 21.0 ± 57 |
| **Cholesterol (mg/dL).** | **34-219** | 95.7 ± 4.2 | 79.5 ± 2.1 |
| **Glucose (mg/dL)** | **46-279** | 129.7 ± 27.8 | 135.0 ± 14.1 |
| **Total Protein (g/dL)** | **3.3-7.6** | 6.3 ± 0.3 | 6.1 ± 0.4 |

**Figure 8** depicts the amino acid sequences of the Heavy and Light chain variable regions of anti-EMP-2 antibodies KS49, KS41, KS89 and KS83 are shown. Suitable CDR sequences of the variable regions are identified using the Kabat CDR definition.

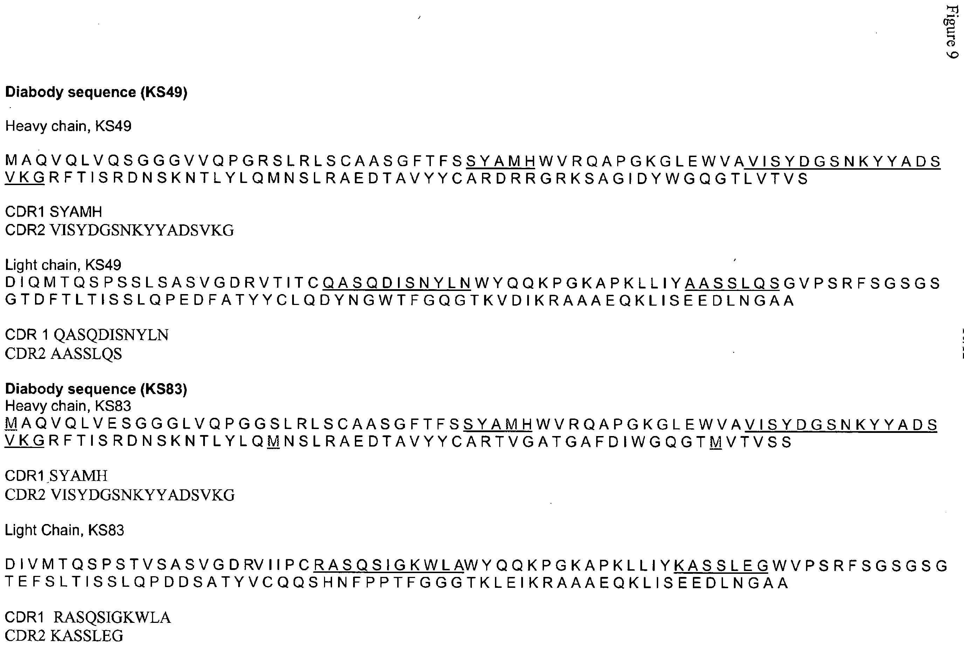

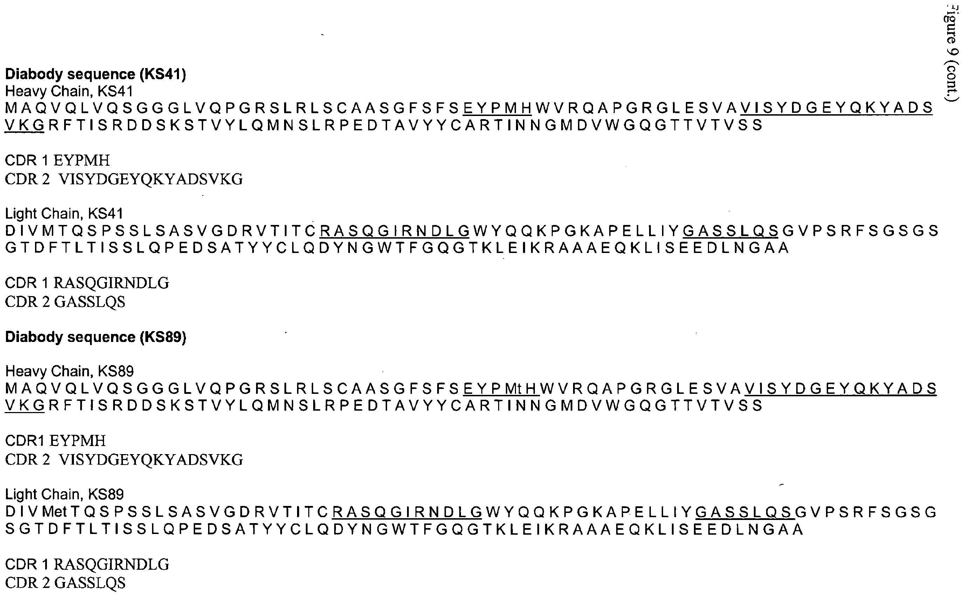

**Figure 9** depicts the amino acid sequences of the Heavy and Light chain variable regions of anti-EMP-2 antibodies KS49, KS41, KS89 and KS83 showing the suitable CDR sequences for use in the antibodies of the invention.

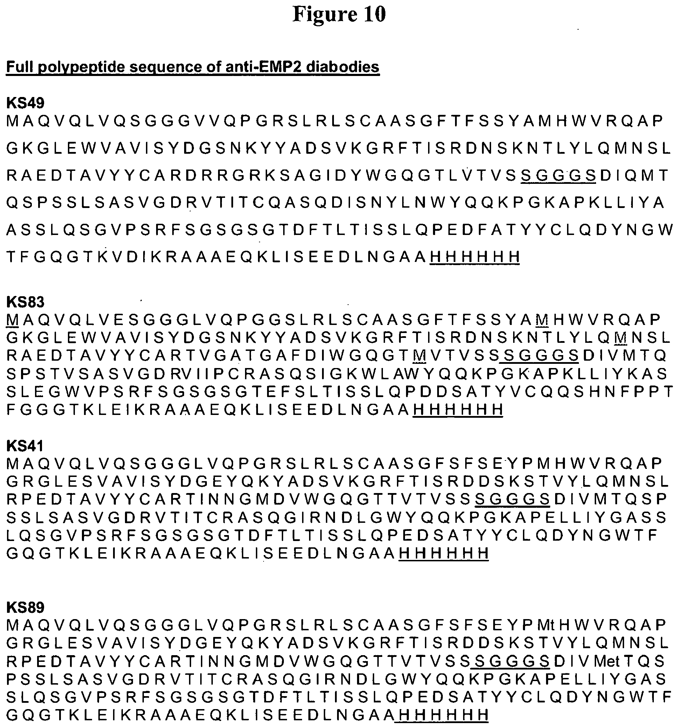

**Figure 10** depicts the amino acid sequences of the KS49, KS41, KS89 and KS83 diabodies with underlining of their linkers and polyhistidine tags.

### DETAILED DESCRIPTION OF THE INVENTION

### Introduction

Breast cancer remains the most common malignancy in women. One particularly aggressive type of breast cancer lacks expression of estrogen-receptor (ER), Her-2/neu and the progesterone receptor (PR). This aggressive type of breast cancer is often referred to as triple negative breast cancer. Triple negative breast cancer accounts for nearly half of all breast cancer deaths. Accordingly, understanding the molecular basis of triple negative breast cancer is necessary to form strategies for detection and treatment.

The Applicants have discovered that EMP2 is highly expressed in over 70% of triple negative breast cancer cases. Methods of diagnosing triple negative breast cancer are known to one of skill in the art. Furthermore, it was previously reported that targeting of EMP2 may offer a therapeutic strategy in treating breast cancer. US Pat. Pub. 20100272732. Accordingly, in its first aspect, the invention provides compositions of anti-EMP2 antibodies and methods of treating triple negative breast cancer. In a specific aspect, the invention provides the administration of anti-EMP2 antibodies in a physiologically acceptable carrier or a pharmaceutically acceptable carrier. In another aspect, the invention provides compositions of anti-EMP2 antibodies and methods of detecting triple negative breast cancer. In another aspect, the invention provides compositions of anti-EMP2 antibodies and methods of coadministration with one or more additional therapies. In another aspect, the invention provides companion diagnostic methods and products for use with the methods and antibodies described herein.

### Antibodies

Antibodies that find use in the present invention can take on a number of formats such as traditional antibodies as well as antibody derivatives, fragments and mimetics. In certain embodiments of this invention, the anti-EMP2 antibodies are KS49, KS41, KS83, or KS89. These antibodies and their use are described herein.

Traditional antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. Heavy chains are classified as mu, delta, gamma, alpha, or epsilon, and define the antibody's isotype as IgM, IgD, IgG, IgA, and IgE, respectively. IgG has several subclasses, including, but not limited to IgG1, IgG2, IgG3, and IgG4. IgM has subclasses, including, but not limited to, IgM1 and IgM2. Thus, "isotype" as used herein is meant any of the subclasses of immunoglobulins defined by the chemical and antigenic characteristics of their constant regions. The known human immunoglobulin isotypes are IgG1, IgG2, IgG3, IgG4, IgA1, IgA2, IgM1, IgM2, IgD, and IgE. It should be understood that therapeutic antibodies can also comprise hybrids of isotypes and/or subclasses.

The amino-terminal portion of each chain includes a variable region of about 100 to 110 or more amino acids primarily responsible for antigen recognition. In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions" that are each 9-15 amino acids long or longer.

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region; Kabat et al., SEQUENCES OF PROTEINS OF IMMUNOLOGICAL INTEREST, 5^{th} Ed. Public Health Service, National Institutes of Health, Bethesda, Md. (1991) and/or those residues forming a hypervariable loop (e.g. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention are described below.

Throughout the present specification, the Kabat numbering system is generally used when referring to a residue in the variable domain (approximately, residues 1-107 of the light chain variable region and residues 1-113 of the heavy chain variable region) (e.g, Kabat *et al*., supra (1991)).

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope. For example, as described herein the antibodies bind to an epitope in the presumptive second extracellular domain of EMP2.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

In some embodiments, the epitope is derived from SEQ ID NO:2, wherein SEQ ID NO:2 is EDIHDKNAKFYPVTREGSYG and represents a 20-mer polypeptide sequence from the second extracellular loop of human EMP2

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and nonconformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning."

The carboxy-terminal portion of each chain defines a constant region primarily responsible for effector function. Kabat *et al.* collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5th edition, NIH publication, No. 91-3242, E.A. Kabat et al).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Another type of Ig domain of the heavy chain is the hinge region. By "hinge" or "hinge region" or "antibody hinge region" or "immunoglobulin hinge region" herein is meant the flexible polypeptide comprising the amino acids between the first and second constant domains of an antibody. Structurally, the IgG CH1 domain ends at EU position 220, and the IgG CH2 domain begins at residue EU position 237. Thus for IgG the antibody hinge is herein defined to include positions 221 (D221 in IgG1) to 236 (G236 in IgG1), wherein the numbering is according to the EU index as in Kabat. In some embodiments, for example in the context of an Fc region, the lower hinge is included, with the "lower hinge" generally referring to positions 226 or 230.

Of interest in the present invention are the Fc regions. By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cγ2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some embodiments, as is more fully described below, amino acid modifications are made to the Fc region, for example to alter binding to one or more FcγR receptors or to the FcRn receptor.

In some embodiments, the antibodies are full length. By "full length antibody" herein is meant the structure that constitutes the natural biological form of an antibody, including variable and constant regions, including one or more modifications as outlined herein.

Alternatively, the antibodies can be a variety of structures, including, but not limited to, antibody fragments, monoclonal antibodies, bispecific antibodies, minibodies, domain antibodies, synthetic antibodies (sometimes referred to herein as "antibody mimetics"), chimeric antibodies, humanized antibodies, antibody fusions (sometimes referred to as "antibody conjugates"), and fragments of each, respectively. Structures that still rely

The antibody may be an antibody fragment. Specific antibody fragments include, but are not limited to, (i) the Fab fragment consisting of VL, VH, CL and CH1 domains, (ii) the Fd fragment consisting of the VH and CH1 domains, (iii) the Fv fragment consisting of the VL and VH domains of a single antibody; (iv) the dAb fragment (Ward et al., 1989, Nature 341:544-546,) which consists of a single variable, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., 1988, Science 242:423-426, Huston et al.,1988, Proc. Natl. Acad. Sci. U.S.A. 85:5879-5883,), (viii) bispecific single chain Fv (WO 03/11161,) and (ix) "diabodies" or "triabodies", multivalent or multispecific fragments constructed by gene fusion (Tomlinson et. al., 2000, Methods Enzymol. 326:461-479; WO94/13804; Holliger et al., 1993, Proc. Natl. Acad. Sci. U.S.A. 90:6444-6448,).

The antibody may be a mixture from different species, e.g. a chimeric antibody and/or a humanized antibody. That is, in the present invention, the CDR sets can be used with framework and constant regions other than those specifically described by sequence herein.

In general, both "chimeric antibodies" and "humanized antibodies" refer to antibodies that combine regions from more than one species. For example, "chimeric antibodies" traditionally comprise variable region(s) from a mouse (or rat, in some cases) and the constant region(s) from a human. "Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536,. "Backmutation" of selected acceptor framework residues to the corresponding donor residues is often required to regain affinity that is lost in the initial grafted construct (US 5530101; US 5585089; US 5693761; US 5693762; US 6180370; US 5859205; US 5821337; US 6054297; US 6407213,). The humanized antibody optimally also will comprise at least a portion of an immunoglobulin constant region, typically that of a human immunoglobulin, and thus will typically comprise a human Fc region. Humanized antibodies can also be generated using mice with a genetically engineered immune system. Roque et al., 2004, Biotechnol. Prog. 20:639-654, entirely incorporated by reference. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein,). Humanization methods include but are not limited to methods described in Jones et al., 1986, Nature 321:522-525; Riechmann et al.,1988; Nature 332:323-329; Verhoeyen et al., 1988, Science, 239:1534-1536; Queen et al., 1989, Proc Natl Acad Sci, USA 86:10029-33; He et al., 1998, J. Immunol. 160: 1029-1035; Carter et al., 1992, Proc Natl Acad Sci USA 89:4285-9, Presta et al., 1997, Cancer Res. 57(20):4593-9; Gorman et al., 1991, Proc. Natl. Acad. Sci. USA 88:4181-4185; O'Connor et al., 1998, Protein Eng 11:321-8,. Humanization or other methods of reducing the immunogenicity of nonhuman antibody variable regions may include resurfacing methods, as described for example in Roguska et al., 1994, Proc. Natl. Acad. Sci. USA 91:969-973,. In one embodiment, the parent antibody has been affinity matured, as is known in the art. Structure-based methods may be employed for humanization and affinity maturation, for example as described in USSN 11/004,590. Selection based methods may be employed to humanize and/or affinity mature antibody variable regions, including but not limited to methods described in Wu et al., 1999, J. Mol. Biol. 294:151-162; Baca et al., 1997, J. Biol. Chem. 272(16):10678-10684; Rosok et al., 1996, J. Biol. Chem. 271(37): 22611-22618; Rader et al., 1998, Proc. Natl. Acad. Sci. USA 95: 8910-8915; Krauss et al., 2003, Protein Engineering 16(10):753-759,. Other humanization methods may involve the grafting of only parts of the CDRs, including but not limited to methods described in USSN 09/810,510; Tan et al., 2002, J. Immunol. 169:1119-1125; De Pascalis et al., 2002, J. Immunol. 169:3076-3084,.

The antibodies of the invention can be multispecific antibodies, and notably bispecific antibodies. These are antibodies that bind to two (or more) different antigens, or different epitopes on the same antigen.

The antibodies may be diabodies.

The antibody may be a minibody. Minibodies are minimized antibody-like proteins comprising a scFv joined to a CH3 domain. Hu et al., 1996, Cancer Res. 56:3055-3061,. In some cases, the scFv can be joined to the Fc region, and may include some or the entire hinge region.

The antibodies of the present invention are generally isolated or recombinant. An "isolated antibody," refers to an antibody which is substantially free of other antibodies having different antigenic specificities. For instance, an isolated antibody that specifically binds to EMP2 is substantially free of antibodies that specifically bind antigens other than EMP2.

An isolated antibody that specifically binds to an epitope, isoform or variant of human EMP2 or murine EMP2 may, however, have cross-reactivity to other related antigens, for instance from other species, such as EMP2 species homologs. Moreover, an isolated antibody may be substantially free of other cellular material and/or chemicals.

Isolated monoclonal antibodies, having different specificities, can be combined in a well defined composition. Thus, for example all possible combinations of the antibodies KS49, KS41, KS83, or KS89 can be combined in a single formulation, if desired.

The following human-origin antibody sequences encode for high-avidity antibodies specific for human (KS49, KS83) and mouse (KS83) EMP2 and have antibody variable region heavy and light chains suitable for use in either aspect of the invention:

### KS49 heavy chain-

M A Q V Q L V Q S G G G V V Q P G R S L R L S C A A S G F T F S S Y A M H
W V R Q A P G K G L E W V A V I S Y D G S N K Y Y A D S V K G R F T I S R D N S K
N T L Y L Q M N S L R A E D T A V Y Y C A R D R R G R K S A G I D Y W G Q G T L
V T V S S

### KS49 light chain-

DIQMTQSPSSLSASVGDRVTITCQASQDISNYLNWYQQ
KPGKAPKLLIYAASSLQSGVPSRFSGSGSGTDFTLTISSLQPE
DFATYYCLQDYNGWTFGQGTKVDIKRAAAEQKLISEEDLNG
AA

### KS83 heavy chain-

M A Q V Q L V E S G G G L V Q P G G S L R L S C A A S G F T F S S Y A M H
W V R Q A P G K G L E W V A V I S Y D G S N K Y Y A D S V K G R F T I S R D N S K
N T L Y L Q M N S L R A E D T A V Y Y C A R T V G A T G A F D I W G Q G T M V T
V S S S

### KS83 light chain-

D I V M T Q S P S T V S A S V G D R V I I P C R A S Q S I G K W L A W Y Q Q K
P G K A P K L L I Y K A S S L E G W V P S R F S G S G S G T E F S L T I S S L Q P D D
S A T Y V C Q Q S H N F P P T F G G G T K L E I K R A A A E Q K L I S E E D L N G A
A

Other diabodies for use according to either aspect of the invention include KS41 and KS89:

### KS41 Heavy Chain-

M A Q V Q L V Q S G G G L V Q P G R S L R L S C A A S G F S F S E Y P M H
W V R Q A P G R G L E S V A V I S Y D G E Y Q K Y A D S V K G R F T I S R D D S K
S T V Y L Q M N S L R P E D T A V Y Y C A R T I N N G M D V W G Q G T T V T V S
S

### KS41 Light Chain-

DIVMTQSPSSLSASVGDRVTITCRASQGIRNDLGWYQQ
KPGKAPELLIYGASSLQSGVPSRFSGSGSGTDFTLTISSLQPE
DSATYYCLQDYNGWTFGQGTKLEIKRAAAEQKLISEEDLNG
AA

### KS89 Heavy Chain-

M A Q V Q L V Q S G G G L V Q P G R S L R L S C A A S G F S F S E Y P M H
W V R Q A P G R G L E S V A V I S Y D G E Y Q K Y A D S V K G R F T I S R D D S K
S T V Y L Q M N S L R P E D T A V Y Y C A R T I N N G M D V W G Q G T T V T V S
S

### KS89 Light Chain-

D I V M T Q S P S S L S A S V G D R V T I T C R A S Q G I R N D L G W Y Q Q
K P G K A P E L L I Y G A S S L Q S G V P S R F S G S G S G T D F T L T I S S L Q P E

D S A T Y Y C L Q D Y N G W T F G Q G T K L E I K R A A A E Q K L I S E E D L N G
A A

Anti-EMP-2 variable region sequences, used to encode proteins on backbones including for native antibody, fragment antibody, or synthetic backbones, can avidly bind EMP-2. Via this binding, these proteins can be used for EMP-2 detection, and to block EMP-2 function. Expression of these variable region sequences on native antibody backbones, or as an scFv, triabody, diabody or minibody, labeled with radionuclide, are particularly useful in in the *in vivo* detection of EMP-2 bearing cells. Expression on these backbones or native antibody backbone are favorable for blocking the function of EMP-2 and/or killing EMP-2 bearing cells (*e*.*g*.gynecologic tumors) *in vivo.*

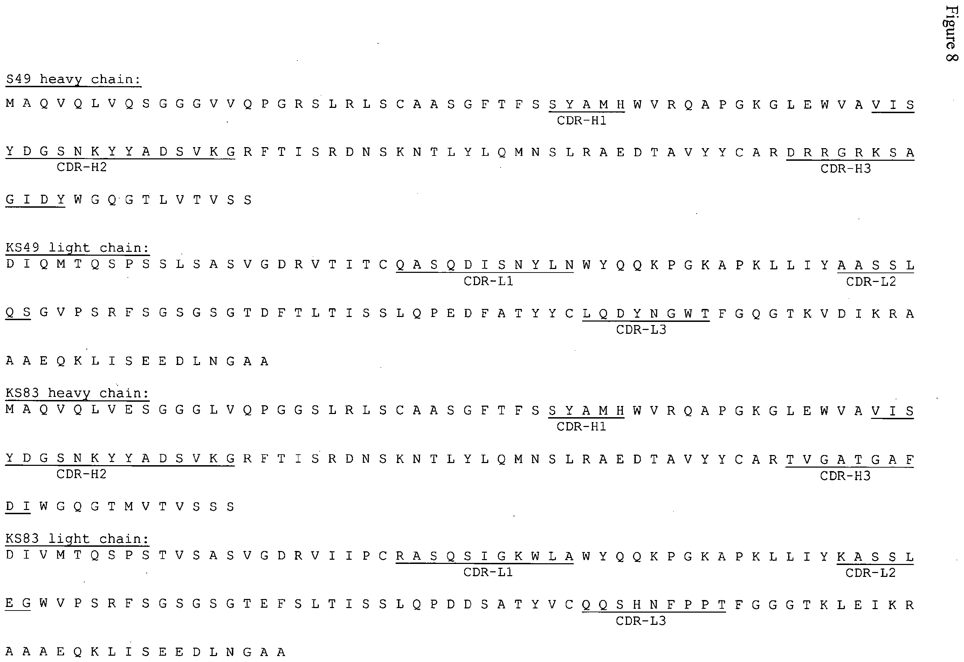

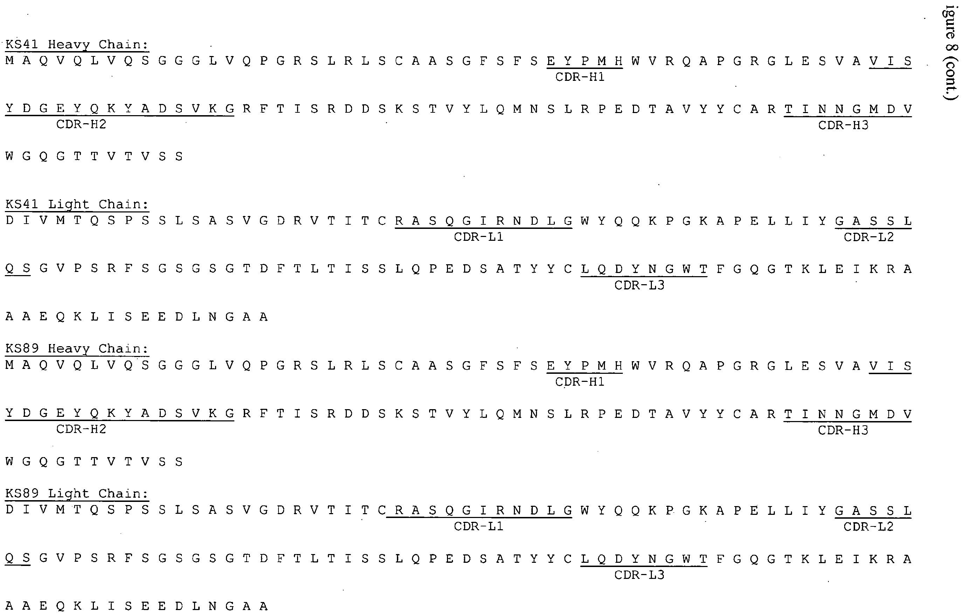

Anti-EMP-2 sequences comprising CDR regions of an antibody selected from KS49, KS83, KS41, and KS89, are shown in Figure 8. The CDR regions provided by the invention may be used to construct an anti-EMP-2 binding protein, including without limitation, an antibody, a scFv, a triabody, a diabody, a minibody, and the like. In a certain embodiments, an anti-EMP-2 binding protein of the invention will comprise at least one CDR region from an antibody selected from KS49, KS83, KS41, and KS89. Anti-EMP-2 binding proteins may comprise, for example, a CDR-H1, a CDR-H2, a CDR-H3, a CDR-L1, a CDR-L2, a CDR-L3, or combinations thereof, from an antibody provided herein. An anti-EMP-2 binding protein may comprise all three CDR-H sequences of an antibody provided herein, all three CDR-L sequences of an antibody provided herein, or both. Anti-EMP2 CDR sequences may be used on an antibody backbone, or fragment thereof, and likewise may include humanized antibodies, or antibodies containing humanized sequences. These antibodies may be used, for example, to detect EMP-2, to detect cells expressing EMP-2 *in vivo,* or to block EMP-2 function. The CDR regions may be defined using the Kabat definition, the Chothia definition, the AbM definition, the contact definition, or any other suitable CDR numbering system.

The CDRs are as follows:
CDR 1 Heavy
SYAMH (49)
SYAMH (83)
EYPMH (41)
EYPMH (89)
CDR 2 Heavy
VISYDGSNKYYADSVKG (49)
VISYDGSNKYYADSVKG (83)
VISYDGEYQKYADSVKG (41)
VISYDGEYQKYADSVKG (89)
CDR 1 Light
QASQDISNYLN (49)
RASQSIGKWLA (83)
RASQGIRNDLG (41)
RASQGIRNDLG (89)
CDR 2 Light
AASSLQS (49)
KASSLEG (83)
GASSLQS (41)
GASSLQS (89)
Diabody sequence (KS49)
Heavy chain, KS49 
M A Q V Q L V Q S G G G V V Q P G R S L R L S C A A S G F T F S S Y
A M H W V R Q A P G K G L E W V A V I S Y D G S N K Y Y A D S V K G R F T I S R
D N S K N T L Y L Q M N S L R A E D T A V Y Y C A R D R R G R K S A G I D Y W G
Q G T L V T V S
CDR1 SYAMH
CDR2 VISYDGSNKYYADSVKG
Light chain, KS49 
DIQMTQSPSSLSASVGDRVTITCQASQDISNYLNW
YQQKPGKAPKLLIYAASSLQSGVPSRFSGSGSGTDFTLTISSL
QPEDFATYYCLQDYNGWTFGQGTKVDIKRAAAEQKLISEED
LNGAA
CDR 1 QASQDISNYLN
CDR2 AASSLQS
Diabody sequence (KS83)
Heavy chain, KS83 
M A Q V Q L V E S G G G L V Q P G G S L R L S C A A S G F T F S S Y
A M H W V R Q A P G K G L E W V A V I S Y D G S N K Y Y A D S V K G R F T I S R
D N S K N T L Y L Q M N S L R A E D T A V Y Y C A R T V G A T G A F D I W G Q G
T M V T V S S
CDR1 SYAMH
CDR2 VISYDGSNKYYADSVKG
Light Chain, KS83 
D I V M T Q S P S T V S A S V G D R V I I P C R A S Q S I G K W L A W Y
Q Q K P G K A P K L L I Y K A S S L E G W V P S R F S G S G S G T E F S L T I S S L Q
P D D S A T Y V C Q Q S H N F P P T F G G G T K L E I K R A A A E Q K L I S E E D L
N G A A
CDR1 RASQSIGKWLA
CDR2 KASSLEG
Diabody sequence (KS41)
Heavy Chain, KS41 
M A Q V Q L V Q S G G G L V Q P G R S L R L S C A A S G F S F S E Y P
M H W V R Q A P G R G L E S V A V I S Y D G E Y Q K Y A D S V K G R F T I S R D D
S K S T V Y L Q M N S L R P E D T A V Y Y C A R T I N N G M D V W G Q G T T V T
V S S
CDR 1 EYPMH
CDR 2 VISYDGEYQKYADSVKG
Light Chain, KS41 
|  D I V M T Q S P S S L S A S V G D R V T I T C R A S Q G I R N D L G W
Y Q Q K P G K A P E L L I Y G A S S L Q S G V P S R F S G S G S G T D F T L T I S S L
Q P E D S A T Y Y C L Q D Y N G W T F G Q G T K L E I K R A A A E Q K L I S E E D
L N G A A
CDR 1 RASQGIRNDLG
CDR 2 GASSLQS
Diabody sequence (KS89)
Heavy Chain, KS89 
M A Q V Q L V Q S G G G L V Q P G R S L R L S C A A S G F S F S E Y P
M t H W V R Q A P G R G L E S V A V I S Y D G E Y Q K Y A D S V K G R F T I S R D
D S K S T V Y L Q M N S L R P E D T A V Y Y C A R T I N N G M D V W G Q G T T V
T V S S
CDR1 EYPMH
CDR 2 VISYDGEYQKYADSVKG
Light Chain, KS89 
D I V Met T Q S P S S L S A S V G D R V T I T C R A S Q G I R N D L G
W Y Q Q K P G K A P E L L I Y G A S S L Q S G V P S R F S G S G S G T D F T L T I S
S L Q P E D S A T Y Y C L Q D Y N G W T F G Q G T K L E I K R A A A E Q K L I S E
E D L N G A A
CDR 1 RASQGIRNDLG
CDR 2 GASSLQS

In some embodiments, the invention provides antibodies (e.g., diabodies, minibodies, triabodies) or fragments thereof having the CDRs of a diabody selected from KS49, KS83, KS41, and KS89. In other embodiments, the diabodies possess the light and heavy chain of a KS49, KS83, KS41, or KS89 diabody. In stillother embodiments, the antibodies are substantially identical in sequence to a diabody selected from the group consisting of KS49, KS83, KS41, and KS89 with or without the polyhistidine tag. In stillother embodiments, the antibodies are substantially identical in sequence to the light and heavy chain sequences of a diabody selected from the group consisting of KS49, KS83, KS41, and KS89. These identities can be 65%, 70%, 75%, 80%, 85%, 90%, and preferably 91%, 92%, 93%, 94%, 95%, 96%, 97%, 98% or 99% or greater amino acid sequence identity. In some further embodiments of any of the above, the antibodies comprise CDRs sequences identical to those of the KS49, KS83, KS41, or KS89 diabody.

The anti-EMP2 antibodies of the present invention specifically bind EMP2 ligands (e.g. the human and murine EMP2 proteins of SEQ ID NOs:1 and 2.

Specific binding for a particular antigen or an epitope can be exhibited, for example, by an antibody having a KD for an antigen or epitope of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the antigen or epitope.

Also, specific binding for a particular antigen or an epitope can be exhibited, for example, by an antibody having a KA or Ka for an antigen or epitope of at least 20-, 50-, 100-, 500-,1000-, 5,000-, 10,000- or more times greater for the epitope relative to a control, where KA or Ka refers to an association rate of a particular antibody-antigen interaction.

### Treatment of breast cancer

### Antibody Compositions for In Vivo Administration

Formulations of the antibodies used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. For example, it may be desirable to provide antibodies with other specificities. Alternatively, or in addition, the composition may comprise a cytotoxic agent, cytokine, growth inhibitory agent and/or small molecule antagonist. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration should be sterile, or nearly so. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and .gamma. ethyl-L-glutamate, nondegradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### Administrative modalities

The antibodies of the invention are administered to a subject, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. In certain aspects, the antibodies and chemotherapeutic agents of the invention are administered to a subject with cancer. In certain aspects, the antibodies of the invention are administered to a subject with breast cancer. In certain aspects, the antibodies of the invention are administered to a subject with triple negative breast cancer. Intravenous or subcutaneous administration of the antibody is preferred.

### Treatment modalities

Therapy is used to provide a positive therapeutic response with respect to a disease or condition. By "positive therapeutic response" is intended an improvement in the disease or condition, and/or an improvement in the symptoms associated with the disease or condition. For example, a positive therapeutic response would refer to one or more of the following improvements in the disease: (1) a reduction in the number of neoplastic cells; (2) an increase in neoplastic cell death; (3) inhibition of neoplastic cell survival; (5) inhibition (i.e., slowing to some extent, preferably halting) of tumor growth; (6) an increased patient survival rate; and (7) some relief from one or more symptoms associated with the disease or condition.

Positive therapeutic responses in any given disease or condition can be determined by standardized response criteria specific to that disease or condition. Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bone scan imaging, endoscopy, and tumor biopsy sampling.

In addition to these positive therapeutic responses, the subject undergoing therapy may experience the beneficial effect of an improvement in the symptoms associated with the disease.

Such a response may persist for at least 4 to 8 weeks, or sometimes 6 to 8 weeks, following treatment according to the methods of the invention. Alternatively, an improvement in the disease may be categorized as being a partial response. By "partial response" is intended at least about a 50% decrease in all measurable tumor burden (i.e., the number of malignant cells present in the subject, or the measured bulk of tumor masses or the quantity of abnormal monoclonal protein) in the absence of new lesions, which may persist for 4 to 8 weeks, or 6 to 8 weeks.

Treatment according to the present invention includes a "therapeutically effective amount" of the medicaments used. A "therapeutically effective amount" refers to an amount effective, at dosages and for periods of time necessary, to achieve a desired therapeutic result.

A therapeutically effective amount may vary according to factors such as the disease state, age, sex, and weight of the individual, and the ability of the medicaments to elicit a desired response in the individual. A therapeutically effective amount is also one in which any toxic or detrimental effects of the antibody or antibody portion are outweighed by the therapeutically beneficial effects.

A "therapeutically effective amount" for tumor therapy may also be measured by its ability to stabilize the progression of disease. The ability of a compound to inhibit cancer may be evaluated in an animal model system predictive of efficacy in human tumors.

Alternatively, this property of a composition may be evaluated by examining the ability of the compound to inhibit cell growth or to induce apoptosis by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

The specification for the dosage unit forms of the present invention are dictated by and directly dependent on (a) the unique characteristics of the active compound and the particular therapeutic effect to be achieved, and (b) the limitations inherent in the art of compounding such an active compound for the treatment of sensitivity in individuals.

The efficient dosages and the dosage regimens for the anti-EMP2 antibodies used in the present invention depend on the disease or condition to be treated and may be determined by the persons skilled in the art.

An exemplary, non-limiting range for a therapeutically effective amount of an anti-EMP2 antibody used in the present invention is about 0.1-100 mg/kg, such as about 0.1-50 mg/kg, for example about 0.1-20 mg/kg, such as about 0.1-10 mg/kg, for instance about 0.5, about such as 0.3, about 1, or about 3 mg/kg. In another embodiment, he antibody is administered in a dose of 1 mg/kg or more, such as a dose of from 1 to 20 mg/kg, e.g. a dose of from 5 to 20 mg/kg, e.g. a dose of 8 mg/kg.

A medical professional having ordinary skill in the art may readily determine and prescribe the effective amount of the pharmaceutical composition required. For example, a physician or a veterinarian could start doses of the medicament employed in the pharmaceutical composition at levels lower than that required in order to achieve the desired therapeutic effect and gradually increase the dosage until the desired effect is achieved.

### Combination Therapy

In some embodiments the anti-EMP2 antibody molecule thereof is used in combination with one or more additional therapeutic agents, e.g. a chemotherapeutic agent. Non-limiting examples of DNA damaging chemotherapeutic agents include topoisomerase I inhibitors (*e*.*g*., irinotecan, topotecan, camptothecin and analogs or metabolites thereof, and doxorubicin); topoisomerase II inhibitors *(e.g.,* etoposide, teniposide, and daunorubicin); alkylating agents (*e*.*g*., melphalan, chlorambucil, busulfan, thiotepa, ifosfamide, carmustine, lomustine, semustine, streptozocin, decarbazine, methotrexate, mitomycin C, and cyclophosphamide); DNA intercalators (*e*.*g*., cisplatin, oxaliplatin, and carboplatin); DNA intercalators and free radical generators such as bleomycin; and nucleoside mimetics *(e.g.,* 5-fluorouracil, capecitibine, gemcitabine, fludarabine, cytarabine, mercaptopurine, thioguanine, pentostatin, and hydroxyurea).

Chemotherapeutic agents that disrupt cell replication include: paclitaxel, docetaxel, and related analogs; vincristine, vinblastin, and related analogs; thalidomide, lenalidomide, and related analogs (*e*.*g*., CC-5013 and CC-4047); protein tyrosine kinase inhibitors (*e.g.,* imatinib mesylate and gefitinib); proteasome inhibitors (*e.g.,* bortezomib); NF-κB inhibitors, including inhibitors of IκB kinase; antibodies which bind to proteins overexpressed in cancers and other inhibitors of proteins or enzymes known to be upregulated, over-expressed or activated in cancers, the inhibition of which downregulates cell replication.

In some embodiments, the antibodies of the invention can be used prior to, concurrent with, or after treatment with any of the chemotherapeutic agents described herein or known to the skilled artisan at this time or subsequently.

### Companion Diagnostics

In other embodiments, this disclsoure relates to companion diagnostic methods and products. In one embodiment, the companion diagnostic method and products can be used to monitor the treatment of breast cancer, specifically triple negative breast cancer, as described herein. In some embodiments, the companion diagnostic methods and products include molecular assays to measure levels of proteins, genes or specific genetic mutations. Such measurements can be used, for example, to predict whether anti-EMP2 therapy will benefit a specific individual, to predict the effective dosage of anti-EMP2 therapy, to monitor anti-EMP2 therapy, adjust anti-EMP2 therapy, tailor the anti-EMP2 therapy to an individual, and track cancer progression and remission.

In some embodiments, the companion diagnostic can be used to monitor a combination therapy.

In some embodiments, the companion diagnostic can include an anti-EMP2 antibody described herein.

In some embodiments, the comapnion diagnostic can be used before, during, or after anti-EMP2 thearpy.

The following references are referred to herein.
1. Elias AD. Triple negative breast cancer: a short review. Am J Clin Oncol. Dec 2010;33(6):637-645.
2. Foulkes WD, Smith IE, Reis-Filho JS. Triple negative breast cancer. N Engl J Med. Nov 11 2010;363(20):1938-1948.
3. Weigelt B, Peterse JL, van 't Veer LJ. Breast cancer metastasis: markers and models. Nat Rev Cancer. Aug 2005;5(8):591-602.
4. Paik S, Kim C, Wolmark N. HER2 status and benefit from adjuvant trastuzumab in breast cancer. NEngl J Med. Mar 27 2008;358(13):1409-1411.
5. Al Saleh S, Sharaf LH, Luqmani YA. Signalling pathways involved in endocrine resistance in breast cancer and associations with epithelial to mesenchymal transition (Review). IntJ Oncol. May 2011;38(5):1197-1217.
6. Al-Ejeh F, Smart CE, Morrison BJ, et al. Breast cancer stem cells: treatment resistance and therapeutic opportunities. Carcinogenesis. May 2011;32(5):650-658.
7. Rivera E, Gomez H. Chemotherapy resistance in metastatic breast cancer: the evolving role of ixabepilone. Breast Cancer Res. 2010;12 Suppl 2:S2.
8. Osborne CK, Schiff R. Mechanisms of endocrine resistance in breast cancer. Annu Rev Med. Feb 18 2011;62:233-247.
9. Pal SK, Childs BH, Pegram M. Triple negative breast cancer: unmet medical needs. Breast Cancer Res Treat. Feb 2011;125(3):627-636.
10. Fu M, Maresh EL, Soslow RA, et al. Epithelial membrane protein-2 is a novel therapeutic target in ovarian cancer. Clin Cancer Res. Aug 1 2010;16(15):3954-3963.
11. Habeeb O, Goodglick L, Soslow RA, et al. Epithelial membrane protein-2 expression is an early predictor of endometrial cancer development. Cancer. Oct 15 2010;116(20):4718-4726.
12. Wadehra M, Natarajan S, Seligson DB, et al. Expression of epithelial membrane protein-2 is associated with endometrial adenocarcinoma of unfavorable outcome. Cancer. Jul 1 2006;107(1):90-98.
13. Morales SA, Mareninov S, Coulam P, et al. Functional consequences of interactions between FAK and epithelial membrane protein 2 (EMP2). Invest Ophthalmol Vis Sci. Oct 2009;50(10):4949-4956.
14. Morales SA, Mareninov S, Prasad P, Wadehra M, Braun J, Gordon LK. Collagen gel contraction by ARPE-19 cells is mediated by a FAK-Src dependent pathway. Exp Eye Res. Dec 2007;85(6):790-798.
15. Morales SA, Mareninov S, Wadehra M, et al. FAK activation and the role of epithelial membrane protein 2 (EMP2) in collagen gel contraction. Invest Ophthalmol Vis Sci. Jan 2009;50(1):462-469.
16. Wadehra M, Forbes A, Pushkarna N, et al. Epithelial membrane protein-2 regulates surface expression of alphavbeta3 integrin in the endometrium. Dev Biol. Nov 15 2005;287(2):336-345.
17. Hakomori SI. Glycosynaptic microdomains controlling tumor cell phenotype through alteration of cell growth, adhesion, and motility. FEBS Lett. May 3 2010;584(9):1901-1906.
18. Shimazaki K, Lepin EJ, Wei B, et al. Diabodies targeting epithelial membrane protein 2 reduce tumorigenicity of human endometrial cancer cell lines. Clin Cancer Res. Nov 15 2008;14(22):7367-7377.
19. van 't Veer LJ, Dai H, van de Vijver MJ, et al. Gene expression profiling predicts clinical outcome of breast cancer. Nature. Jan 31 2002;415(6871):530-536.
20. Obermayr E, Sanchez-Cabo F, Tea MK, et al. Assessment of a six gene panel for the molecular detection of circulating tumor cells in the blood of female cancer patients. BMC Cancer. 2010;10:666.
21. Kao J, Salari K, Bocanegra M, et al. Molecular profiling of breast cancer cell lines defines relevant tumor models and provides a resource for cancer gene discovery. PLoS One. 2009;4(7):e6146.
22. Shimazaki K, Wadehra M, Forbes A, et al. Epithelial membrane protein 2 modulates infectivity of Chlamydia muridarum (MoPn). Microbes Infect. Jul 2007;9(8):1003-1010.
23. Wei WZ, Shi WP, Galy A, et al. Protection against mammary tumor growth by vaccination with full-length, modified human ErbB-2 DNA. Int J Cancer. May 31 1999;81(5):748-754.
24. Habeeb O, Goodglick L, Soslow RA, et al. Epithelial membrane protein-2 expression is an early predictor of endometrial cancer development. Cancer. Jun 24 2010.
25. Shimazaki K, Chan AM, Moniz RJ, et al. Blockade of epithelial membrane protein 2 (EMP2) abrogates infection of Chlamydia muridarum murine genital infection model. FEMS Immunol Med Microbiol. Mar 2009;55(2):240-249.
26. Xuan C, Steward KK, Timmerman JM, Morrison SL. Targeted delivery of interferon-alpha via fusion to anti-CD20 results in potent antitumor activity against B-cell lymphoma. Blood. Apr 8 2010;115(14):2864-2871.
27. Helguera G, Penichet ML. Antibody-cytokine fusion proteins for the therapy of cancer. Methods Mol Med. 2005;109:347-374.
28. Qu Z, Griffiths GL, Wegener WA, et al. Development of humanized antibodies as cancer therapeutics. Methods. 2005;36(1):84-95.
29. Morrison SL, Wims L, Wallick S, Tan L, Oi VT. Genetically engineered antibody molecules and their application. Ann N Y Acad Sci. 1987;507:187-198.
30. Yoon NK, Maresh EL, Elshimali Y, et al. Elevated MED28 expression predicts poor outcome in women with breast cancer. BMC Cancer. 2010;10:335.
31. Yoon NK, Maresh EL, Shen D, et al. Higher levels of GATA3 predict better survival in women with breast cancer. Hum Pathol. Dec 2010;41(12):1794-1801.
32. Yoon NK, Seligson DB, Chia D, et al. Higher expression levels of 14-3-3sigma in ductal carcinoma in situ of the breast predict poorer outcome. Cancer Biomark. 2009;5(4):215-224.
33. Shen D, Nooraie F, Elshimali Y, et al. Decreased expression of annexin A1 is correlated with breast cancer development and progression as determined by a tissue microarray analysis. Hum Pathol. Dec 2006;37(12):1583-1591.
34. Mah V, Seligson DB, Li A, et al. Aromatase expression predicts survival in women with early-stage non small cell lung cancer. Cancer Res. Nov 1 2007;67(21):10484-10490.
35. Presson AP, Yoon NK, Bagryanova L, et al. Protein expression based multimarker analysis of breast cancer samples. BMC Cancer. 2011;11:230.
36. Rakha EA, E1-Sayed ME, Green AR, Lee AH, Robertson JF, Ellis IO. Prognostic markers in triple negative breast cancer. Cancer. Jan 1 2007;109(1):25-32.
37. Subik K, Lee JF, Baxter L, et al. The Expression Patterns of ER, PR, HER2, CK5/6, EGFR, Ki-67 and AR by Immunohistochemical Analysis in Breast Cancer Cell Lines. Breast Cancer (Auckl). 2010;4:35-41.
38. Holliger P, Prospero T, Winter G. "Diabodies": small bivalent and bispecific antibody fragments. Proc Natl Acad Sci U S A. Jul 15 1993;90(14):6444-6448.
39. Fu M, Rao R, Sudhakar D, et al. Epithelial Membrane Protein-2 Promotes Endometrial Tumor Formation through Activation of FAK and Src. PLoS One. 2011;6(5):e19945.
40. Haffty BG, Yang Q, Reiss M, et al. Locoregional relapse and distant metastasis in conservatively managed triple negative early-stage breast cancer. J Clin Oncol. Dec 20 2006;24(36):5652-5657.
41. Wadehra M, Forbes A, Pushkarna N, et al. Epithelial membrane protein-2 regulates surface expression of alphavbeta3 integrin in the endometrium. DevBiol. 2005;287:336-345.
42. Wadehra M, Iyer R, Goodglick L, Braun J. The tetraspan protein epithelial membrane protein-2 interacts with beta1 integrins and regulates adhesion. J Biol Chem. Oct 25 2002;277(43):41094-41100.
43. Huang T, Fu M, Kuga D, et al. EMP2 regulates FAK signaling and contributes to pathogenesis of Glioblastoma Multiforme. submited. 2010.
44. Hochgrafe F, Zhang L, OToole SA, et al. Tyrosine phosphorylation profiling reveals the signaling network characteristics of Basal breast cancer cells. Cancer Res. Nov 15 2010;70(22):9391-9401.
45. Schaller MD, Frisch SM. PND-1186 FAK inhibitor selectively promotes tumor cell apoptosis in three-dimensional environments. Cancer Biol Ther. May 2010;9(10):791-793.
46. Pylayeva Y, Gillen KM, Gerald W, Beggs HE, Reichardt LF, Giancotti FG. Ras- and PI3K-dependent breast tumorigenesis in mice and humans requires focal adhesion kinase signaling. J Clin Invest. Feb 2009;119(2):252-266.
47. Dahlin K, Mager EM, Allen L, et al. Identification of genes differentially expressed in rat alveolar type I cells. Am J Respir Cell Mol Biol. Sep 2004;31(3):309-316.
48. Wadehra M, Sulur GG, Braun J, Gordon LK, Goodglick L. Epithelial membrane protein-2 is expressed in discrete anatomical regions of the eye. Exp Mol Pathol. Apr 2003;74(2):106-112.

The invention is now described with reference to the following examples. These examples are provided for the purpose of illustration only and the invention should in no way be construed as being limited to these examples but rather should be construed to encompass any and all variations which become evident as a result of the teachings provided herein.

### EXAMPLES

### EXAMPLE 1: EMP2 protein expression in cancerous breast tissue

A study was conducted to examine the expression level of EMP2 in breast cancer. Initially, a Western blot analysis and immunohistochemistry (IHC) of a set of 5 flash frozen human invasive ductal carcinoma samples and 3 samples of normal glandular breast epithelium was conducted.

In order to conduct this analysis breast cancer cell lines or tissue were lysed in Laemmli buffer, and for EMP2 detection, were treated with N-glycosidase F (New England Biolabs, Beverly, MA) to remove N-link glycosylation. Proteins were separated using SDS-PAGE, transferred onto nitrocellulose membrane and blocked in 10% nonfat dry milk in TBS-Tween20 buffer. Blots were probed using rabbit anti-human EMP2 antisera (1:2000). Proteins were then detected by using a horseradish peroxidase conjugated goat anti¬rabbit antibody. As a loading control, β-actin expression was detected using primary monoclonal anti-β actin (Sigma) and secondary horseradish peroxidase-conjugated goat anti-mouse IgG (Amersham™, Piscataway, NJ). Bands were visualized using ECL™ detection reagents (Amersham™).

As shown in Figure 1A, consistently, expression of EMP2 was robust in breast cancer samples compared to weak or non-detectable levels in non-malignant glandular epithelium. EMP2 was predominantly present at the membrane and/or cytoplasm of the malignant breast epithelium (Figure 1B).

### EXAMPLE 2: Expression Profiled of EMP2 by Tissue Microarray

The expression profile of EMP2 in human breast cancer on a population basis was examined using tissue microarray (TMA) technology. A high density breast cancer TMA was constructed using archival breast tissue samples from 212 patients who had breast surgery at the UCLA Medical Center between 1995 and 2000. Samples were collected as approved and monitored by the UCLA Institutional Review Board. The demographic, clinical, and pathology characteristics are shown in Table 2. Surgical samples from each patient often yielded more than one histology.

**Table 2**

| **Clinical Parameter** | **n** |
|---|---|
| **Total Number of Patients** | **74** |
| **Age (≥ 55)** | **32** |
| **ER+** | **53** |
| **PR+** | **52** |
| **Her2+** | **18** |
| | |
| **Invasive Ductal Carcinoma** | **53** |
| **Invasive Lobular Carcinoma** | **4** |
| **Other diagnosis** | **17** |
| | |
| **Grade 1** | **19** |
| **Grade 2** | **22** |
| **Grade 3** | **30** |
| **Stage I** | **26** |
| **Stage II** | **31** |
| **Stage HI** | **16** |
| **Stage IV** | **1** |
| | |
| **Tumor Size (≥ 2.5cm)** | **35** |
| **Lymph node metastasis** | **30** |
| **Lympho-vasculature invasion** | **24** |
| | |
| **Recurrence** | **8** |
| **Disease-related death** | **13** |

This study focused on the following categories: normal glandular/ductal epithelium (n=139 spots), ductal hyperplasia (DH; n=35 spots), ductal carcinoma in situ (DCIS; n = 142 spots), invasive ductal carcinoma (IDC; n = 236 spots), and lymph node metastatic lesions (n = 69 spots).

A semiquantitative integrated scoring of the intensity and frequency of EMP2 staining was performed by a pathologist (RAS). The following formula was used to derive the integrated value: [3(%a) + 2(%b) + 1(%c]/100, where a, b, and c is the percentage of cells staining at intensity 3, 2, and 1, respectively.

Compared to non-malignant glandular and ductal mammary epithelium there was a significant increase in EMP2 expression in DCIS, invasive carcinoma and lymph node metastatic lesion (Figure 1C).

On the TMA, there were 11 cases of triple negative breast cancer "TNBC." TNBC tumors lack estrogen receptor (ER), progesterone receptor (PR) and Her-2/neu expression. Of these 11 cases, 8 (73%) had relatively high expression levels of EMP2 while 3 cases showed relatively lower levels of expression.

An additional set of 23 TNBC cases were also examined for EMP2 expression. Similar to above, 17 of these 23 cases of TNBC were positive for EMP2.

**Table 3**

| **EMP 2 Expression** | **Number of Cases** |
|---|---|
| Below the level of detection | 1 |
| Weak | 5 |
| Strong | 17 |

### EXAMPLE 3: Effect of anti-EMP2 Treatment In Vitro

To test the effects of anti-EMP2 treatment on breast cancer cells, the following breast cancer cell lines were utilized MDA-MB-231, MDA-MB-468, BT-20, and HS578, which are triple negative for ERα, PR and Her-2/neu expression and ZR-75-1, which expresses these three receptors.

Human breast cancer cell lines HS578 (a gift from Dr. John Colicelli, UCLA), BT-20, MDA-MB-231 (both gifts of Dr. Richard Pietras, UCLA), ZR-75-1 and MDA-MB-468 (American Type Culture Collection; ATCC, Manassas, VA) were cultivated in DMEM medium (Mediatech, Manassas, VA) supplemented with 10% fetal calf serum (Hyclone Laboratories, Logan, UT), 2 mM L-glutamine, 1 mM sodium pyruvate, 100 U/ml penicillin, and 100 U/ml streptomycin (all from Invitrogen Life Technologies, Carlsbad, CA).

Cells were cultured at 37°C in a humidified 5% CO2. Additionally, HS578 sublines were prepared which overexpress a EMP2 have been previously described. Control HS578 were also produced. These sublines were termed HS578/EMP2 and HS578/V, respectively. In addition, the murine mammary tumor D2F2 cells were utilized and maintained in RPMI medium supplemented as above. D2F2 cells (syngeneic for BALB/c mice) were a kind gift from Dr. Wei-Zen Wei (Wayne State University, Detroit, MI). Western blot analysis was used to confirm the EMP2 expression levels in each cell line

Each cell line was tested for EMP2 expression; a representative Western blot is shown in Figure 1D. EMP2 is present in 4 out of 5 cell lines but below the level of detection in HS578 cells. A variant of HS578 which expressed EMP2 (HS578/EMP2) or, as a control, infected with vector plus GFP alone (HS578/V) was also constructed. The expression of EMP2 in HS578/EMP2 is shown in Figure 1D. We also detected the expression of EMP2 in the murine mammary tumor cell line D2F2 and constructed a variant of D2F2 which overexpressed EMP2 coupled to a FLAG tag (D2F2/EMP2). Mouse spleenocytes was been used as a negative control (Figure 1E).

For both *in vitro* and *in vivo* (described below) targeting a recombinant anti-EMP2 diabody which recognizes conserved regions of both human and murine EMP2 was utilized. In addition a full length humanized anti-EMP2 antibody (IgG1) was also constructed and utilized in these targeting experiments.

In order to construct the full length human anti-EMP2 antibody (IgG1) based on a previously described anti-EMP2 diabody construct, KS83. To accomplish this, the diabody variable (V) region sequence was obtained by PCR then cloned into pCR-II-TOPO vector (Invitrogen). This construct included a sequence encoding a function signal peptide for proper secretion. The cloning was confirmed by sequencing. The sequence encoding the EMP2 variable light (VL) and heavy chain regions (VH) were inserted into the κ light chain and γ heavy chain IgG1 expression vector, respectively. These vectors contain the cytomegalovirus promoter (CMV) and have been shown to secrete functional recombinant antibodies in murine myeloma cells. The heavy and light chain expression vectors were transfected into the murine myeloma cell line Sp2/0-Ag14 as described previously. Cells were subcloned then screened by ELISA (described below) using goat anti-human IgG (Invitrogen) and goat anti-human κ chain (Sigma-Aldrich). The five highest producing subclones were isolated to use for S-35 biosynthetic antibody labeling and immunoprecipitation with hyperimmune rabbit antihuman IgG (Sigma-Aldrich) and Staph A (IgGSorb, The Enzyme Center, Malden, MA), to validate and select the optimal clone. Once selected, cells were expanded into roller bottles to maximize the secretion of antibodies. After 2-3 weeks, supernatants were collected and filtered for purification as below.

Supernatants were passed over a 1.5 mL volume FlexColumn (Thermo Fisher Scientific) with 1 mL of protein A-Sepharose™ (Sigma-Aldrich), and bound proteins were eluted with 2 column volumes of 0.2 M citrate buffer (pH 4.5), 3 column volumes of 0.1 M glycine-HCl (pH 2.5) and 2 column volumes of 0.1 M glycine-HCk (pH 2.0), sequentially. The eluted fractions containing the desired antibodies were dialyzed against PBS with Slide-A-Lyzer® Dialysis Cassettes (Thermo Fisher Scientific). The final concentration of purified antibodies was measured with Nanodrop 2000 (Thermo Scientific). Binding specificity of the recombinant antibody was determined by ELISA and flow cytometry (see below).

For ELISA analysis biotinylated 24 amino acid peptides corresponding to the extracellular loop of human EMP2 were coated onto streptavidin-coated 96-well plates (Roche Applied Science, Indianapolis, IN). Specifically, bound antibodies were detected with HRP conjugated goat anti-human IgG (Jackson Immunoresearch, West Grove, PA) and TMB solution (eBioscience, San Diego, CA). Absorbance at 450 nm was determined using a microplate reader Model 550 (Bio-Rad, Hercules, CA).

For FACS analysis EMP2-positive cells (HEC1a/EMP2, HEC1a, or D2F2 cells) or EMP2-negative cells (EL4 or Ramos) were resuspended at a concentration of 10⁶ cells in 1 mL of cold PBS+0.2% BSA buffer (flow buffer). The cell suspension was centrifuged for 5 min at 500g, at 4oC. Cells were then incubated with 1 µg of recombinant anti-EMP2 IgG1 for 2 h at 4°C. An IgG specific for the hapten dansyl, 5-dimethylamino naphthalene-1-sulfonyl chloride (DNS) was used as a non-targeted antibody negative control. Cells were washed three times and then incubated for 30 min at 4°C with PE-conjugated goat anti-human IgG (Jackson Immunoresearch). Cells were washed and resuspended in flow buffer. Flow cytometry was immediately performed with a Becton Dickinson FACScan Analytic Flow Cytometer (Becton Dickinson) in the UCLA Jonsson Comprehensive Cancer Center and Center for AIDS Research Flow Cytometry Core Facility.

Previous studies have shown that EMP2 expression promotes invasion through FAK and Src activation. To determine if EMP2 antibodies inhibit invasion, cells were treated with anti-EMP2 KS83 or full length KS83-IgG1 or the appropriate controls. Two hour treatment with either EMP2 specific diabodies or full length KS83-IgG1 inhibited transwell invasion compared to the controls (Figure 2A, B). Without being bound by theory, the apparent mechanism behind this action, is an EMP2 antibodies mediated reduced in Src phosphorylation (Figure 2C, D). A modest reduction in FAK phosphorylation was also observed.

Although treatment of EMP2-expressing endometrial or ovarian cancer cells with the anti-EMP2 diabody results in cell death, such an effect has not been tested in breast cancer cells. In order to test cellular viability in the presence anti-EMP2 diabody KS83, control diabody A10, anti-EMP2 recombinant IgG1, or control IgG1 (Sigma-Aldrich), 5 x 10⁴ ZR-75-1, MDA-MB-468, HS578/EMP2, or HS578/V cells were incubated *in vitro* with molar equivalent amounts of anti-EMP2 diabody (KS83), non-immune diabody (A10), full length KS83-IgG1, or a saline control for 24-96 hours. Cells were then harvested and the percentage of non-viable cells was determined using trypan blue exclusion..

Treatment of ZR-75-1, MDA-MB-468, and HS578/EMP2 cells with either KS83 diabody or full length KS83-IgG1 resulted in ~35%-50% loss of viability compared to the non-immune A10 diabody or saline control (Figure 3A,B). Treatment of HS578/V (EMP2-negative) cells with the diabodies or full length KS83-IgG1 caused no loss in viability indicating that the effect was dependent on EMP2 expression (Figure 3A).

The cell death observed apparently occurs via an apoptotic mechanism. Cells incubated with KS83 diabody or full length KS83-IgG1 (or appropriate controls) were stained with the apoptosis marker, annexin V, as well as the viability marker propidium iodide. Cells were harvested and stained with an Annexin V-FITC detection kit according to manufacturer's instructions (BD Biosciences). As shown in Figure 3B, incubation of ZR-75-1 or MDA-MB-468 cells with anti-EMP2 diabody or full length KS83-IgG1, induced an increase in apoptosis within 48 hours (Figure 3C). Such an effect was not seen with control reagents.

### EXAMPLE 4: Effect of anti-EMP2 Treatment In Vivo

The effectiveness of anti-EMP2 immune reagents to limit tumor growth in a mouse model system was tested.

At the outset, the *in vivo* toxicity of KS83 diabodies and full length KS83 IgG₁ was assessed. 7-week-old female wild-type (C57BL/6) mice obtained from Jackson Laboratories. Mice were maintained in accordance with the National Academy of Science Guide for the Care and Use of Laboratory Animals in the Vivarium of UCLA. At least three animals per group were injected intravenously (i.v.) weekly with increasing concentrations (0.5-5 mg/kg) of anti-EMP2 IgG1 antibody or a vehicle control (sterile PBS) for 7 weeks. Serum was collected every 14 days. At the end of the time course, mice were euthanized by cervical dislocation. Tissues (kidney, liver, spleen, lung, uterus, heart, ovary, and skin) were collected fixed in formalin, processed, embedded in paraffin, sectioned, stained with Hematoxylin and Eosin, and analyzed for pathological changes by a pathologist. Complete blood counts and liver enzyme analysis (serum alanine aminotransferase, direct and total bilirubin) were quantified by the UCLA Medical Center Clinical Laboratories.

Weekly injections of KS83 or control diabodies demonstrated no detectable toxicity or adverse affects in mice. Likewise, upon extensive testing with twice weekly injections of the full length anti-EMP2 IgG1 in BALB/c mice (10 mg/kg) for seven weeks, no indication of systemic or tissue-specific damage or toxicity was observed (Figure 7).

For a preclinical xenograft model of antibody directed therapy for breast cancer, the triple negative cell line, MDA-MB-468, and the anti-EMP2 diabody KS83 or the full-length anti-EMP2 IgG1 were utilized.

To create tumor xenograft models, 4-6-week old female BALB/c nude mice (Charles River, MA) were used for each condition. Mice were maintained in accordance with the National Academy of Science Guide for the Care and Use of Laboratory Animals in the vivarium of UCLA. Briefly, 5x106 MDA-MB-468 or 2x107 Ramos cells suspended in 5% BD matrigel™ (BD Biosciences) were injected subcutaneously (s.c.) into the shoulder of female athymic mice. Tumor volume was calculated with the formula: length x width²/2. When tumors became detectable (day 0), they were injected intratumor (i.t.) with 1 mg/kg dose of anti-EMP2 diabodies KS83 or non-immune diabody A10 twice a week. Alternatively, the tumors were injected with 3 mg/kg i.t. or between 1-10mg/kg systemically with anti-EMP2 IgG1 or control IgG (Sigma) weekly. Tumor size was monitored, and mice euthanized once tumors approached 1.5 cm in diameter or became ulcerated. Tumors were isolated, fixed and processed for hematoxylin and eosin staining.

As shown in Figure 4A, treatment of tumors with KS83 or the full length anti-EMP2 IgG1 resulted in significant reduction of tumor growth by day 15 compared to non-immune reagent treatment. Upon histological examination, tumors treated with anti-EMP2 reagents showed wide areas of necrosis in contrast with tumors treated with non-immune reagents (Figure 4B).

Systemic injections of anti-EMP2 IgG1 as a therapeutic approach was also tested. Similar to i.t. injection, systemic treatment with full length anti-EMP2 IgG1 significantly reduced tumor growth compared to treatment with control IgG (Figure 4C; day 28). This model also produced extensive necrosis in response to anti-EMP2 IgG treatment (Figure 4D). Comparatively, reduced areas of necrosis were observed in the control IgG treated group compared with the anti-EMP2 IgG1 treated group. To validate that the effects of anti-EMP2 IgG1 treatment were specific, the B lymphoma cell line Ramos was used as a control. Ramos cells do not express EMP2. Injections twice a week of either anti-EMP2 IgG1 or Ctrl IgG1 showed no difference in tumor load (Figure 4E) or in tumor histology (Figure 4F).

As a further confirmation of the efficacy of anti-EMP2 IgG1 treatment, murine mammary D2F2 cells were utilized. D2F2 cells express EMP2 on the plasma membrane and are syngeneic to BALB/c mice. Susceptibility of D2F2 cells to anti-EMP2 IgG1 treatment was determined through titration of antibody and measurement of apoptosis and cell death by flow cytometry (Figure 5A). A dose dependent increase in cell death relative to antibody concentration was observed, and the effects were significantly increased compared to treatment with the control IgG.

To determine if this effect could be *recapitulated in vivo,* 1x10⁵ D2F2 tumors were injected into the mammary pad of BALB/c mice (Charles River). Once tumors were detectable, they were treated twice with 10 mg/kg of anti-EMP2 IgG1 or control IgG. After the final point, mice were euthanized and tumors isolated as above. While these tumors grow very rapidly, two treatments within the week of anti-EMP2 IgG1 significantly reduced tumor load by 50% compared to control IgG treatment (Figure 5B) with tumors exhibiting pronounced necrosis (Figure 5C).

For statistical analysis, differences *in in vitro* phenotypic changes or *in vivo* tumor growth were evaluated using a two-tailed Student's unpaired t-test at a 95% confidence level (GraphPad Prism version 3.0; GraphPad Software, La Jolla, CA). P values <0.05 were considered significant. TMA analyses were performed as previously described (30-34) with the Mann-Whitney test for two-group comparisons. A P value < 0.05 was considered significant.

## Claims

1. An antibody that specifically binds to an epitope in the second extracellular loop of EMP2 wherein the epitope comprises the amino acid sequence DIHDKNAKFYPVTREGSYG for use in the treatment of breast cancer wherein the breast cancer comprises a triple negative breast cancer tumor.

2. The antibody for use according to claim 1, wherein the antibody further comprises a physiological acceptable carrier or a pharmaceutically acceptable carrier.

3. The antibody for use according to claim 1, wherein the antibody:
i) competes with an antibody comprising the heavy and light chain variable regions of a KS49, a KS41, a KS83, or a KS89 diabody;
ii) shares 90% amino acid identity with heavy and light chain variable regions of a KS49, a KS41, a KS83, or a KS89 diabody; and/or
iii) comprises CDR sequences identical to those of a KS49, a KS41, a KS83, or a KS89 diabody.

4. A composition comprising an antibody according to any one of claims 1 to 3 and
i) at least one additional anti-cancer agent; and/or
ii) a companion diagnostic,
for use in the treatment of breast cancer wherein the breast cancer comprises a triple negative breast cancer tumor.

5. The composition for use according to claim 4, wherein the at least one additional anti-cancer agent:
i) is selected from the group consisting of platinum-based chemotherapy drugs, taxanes, tyrosine kinase inhibitors, anti-EGFR antibodies, anti-ErbB2 antibodies, and combinations thereof;
ii) comprises an EGFR inhibitor;
iii) comprises a VEGF inhibitor.

6. The composition for use according to claim 5, wherein the EGFR inhibitor:
i) comprises an anti-EGFR antibody;
ii) is a small molecule inhibitor of EGFR signaling.

7. The composition for use according to claim 6, wherein the anti-EGFR antibody:
i) comprises cetuximab;
ii) is selected from the group consisting of matuzumab, panitumumab, and nimotuzumab.

8. The composition for use according to claim 6, wherein the small molecule inhibitor of EGFR signaling is selected from the group consisting of gefitinib, lapatinib, canertinib, pelitinib, erlotinib HCL, PKI-166, PD158780, and AG 1478.

9. The composition for use according to claim 5, wherein the VEGF inhibitor comprises an anti-VEGF antibody such as bevacizumab.

10. The composition for use according to claim 4, wherein the companion diagnostic comprises an anti-EMP2 antibody.

11. The antibody or composition for use according to any one of claims 1 to 10 wherein the antibody is conjugated with an effector moiety.

12. The antibody or composition for use according to claim 11, wherein the effector moiety is a toxic agent such as ricin.

13. The antibody or composition for use according to any one of claims 1 to 12 wherein the treatment comprises blocking invasiveness of the cancer.

14. The antibody or composition for use according any one of claims 1 to 13, wherein the antibodies are used in vaccine therapies for the cancer.

15. The antibody or composition for use according any one of claims 1 to 14, wherein the treatment is of a patient which is a mammal.

16. The antibody or composition for use according any one of claims 1 to 15, wherein the treatment is of a patient which is a human.

## Patentansprüche

1. Antikörper, der sich spezifisch an ein Epitop in der zweiten extrazellulären Schleife von EMP2 bindet, wobei das Epitop die Aminosäuresequenz DIHDKNAKFYPVTREGSYG zur Verwendung bei der Behandlung von Brustkrebs umfasst, wobei der Brustkrebs einen triple-negativen Brustkrebstumor umfasst.

2. Antikörper zur Verwendung nach Anspruch 1, wobei der Antikörper ferner einen physiologisch unbedenklichen Träger oder einen pharmazeutisch unbedenklichen Träger umfasst.

3. Antikörper zur Verwendung nach Anspruch 1, wobei der Antikörper:
i) mit einem Antikörper konkurriert, der die variablen Regionen einer schweren und leichten Kette eines KS49-, eines KS41-, eines KS83- oder eines KS89-Diabodys umfasst;
ii) 90 % Aminosäureidentität mit variablen Regionen einer schweren und leichten Kette eines KS49-, eines KS41-, eines KS83- oder eines KS89-Diabodys teilt; und/oder
iii) CDR-Sequenzen umfasst, die mit denen eines KS49-, eines KS41-, eines KS83- oder eines KS89-Diabodys identisch sind.

4. Zusammensetzung, umfassend einen Antikörper nach einem der Ansprüche 1 bis 3 und
i) mindestens ein zusätzliches Antikrebsmittel; und/oder
ii) ein Begleitdiagnostikum,
zur Verwendung bei der Behandlung von Brustkrebs, wobei der Brustkrebs einen triple-negativen Brustkrebstumor umfasst.

5. Zusammensetzung zur Verwendung nach Anspruch 4, wobei das mindestens eine zusätzliche Antikrebsmittel:
i) aus der Gruppe bestehend aus platinbasierten Chemotherapeutika, Taxanen, Tyrosinkinase-Inhibitoren, Anti-EGFR-Antikörpern, Anti-ErbB2-Antikörpern und Kombinationen davon ausgewählt wird;
ii) einen EGFR-Inhibitor umfasst;
iii) einen VEGF-Inhibitor umfasst.

6. Zusammensetzung zur Verwendung nach Anspruch 5, wobei der EGFR-Inhibitor:
i) einen Anti-EGFR-Antikörper umfasst;
ii) ein niedermolekularer Inhibitor der EGFR-Signalisierung ist.

7. Zusammensetzung zur Verwendung nach Anspruch 6, wobei der Anti-EGFR-Antikörper:
i) Cetuximab umfasst;
ii) aus der Gruppe bestehend aus Matuzumab, Panitumumab und Nimotuzumab ausgewählt wird.

8. Zusammensetzung zur Verwendung nach Anspruch 6, wobei der niedermolekulare Inhibitor der EGFR-Signalisierung aus der Gruppe bestehend aus Gefitinib, Lapatinib, Canertinib, Pelitinib, Erlotinib HCL, PKI-166, PD158780 und AG 1478 ausgewählt wird.

9. Zusammensetzung zur Verwendung nach Anspruch 5, wobei der VEGF-Inhibitor einen Anti-VEGF-Antikörper wie Bevacizumab umfasst.

10. Zusammensetzung zur Verwendung nach Anspruch 4, wobei das Begleitdiagnostikum einen Anti-EMP2-Antikörper umfasst.

11. Antikörper oder Zusammensetzung zur Verwendung nach einem der Ansprüche 1 bis 10, wobei der Antikörper mit einer Effektoreinheit konjugiert ist.

12. Antikörper oder Zusammensetzung zur Verwendung nach Anspruch 11, wobei die Effektoreinheit ein toxisches Mittel wie Ricin ist.

13. Antikörper oder Zusammensetzung zur Verwendung nach einem der Ansprüche 1 bis 12, wobei die Behandlung das Blockieren der Invasivität des Krebses umfasst.

14. Antikörper oder Zusammensetzung zur Verwendung nach einem der Ansprüche 1 bis 13, wobei die Antikörper in Impfstofftherapien für den Krebs verwendet werden.

15. Antikörper oder Zusammensetzung zur Verwendung nach einem der Ansprüche 1 bis 14, wobei die Behandlung einen Patienten betrifft, der ein Säugetier ist.

16. Antikörper oder Zusammensetzung zur Verwendung nach einem der Ansprüche 1 bis 15, wobei die Behandlung einen Patienten betrifft, der ein Mensch ist.

## Revendications

1. Anticorps qui se lie spécifiquement à un épitope dans la seconde boucle extracellulaire de l'EMP2, où l'épitope comprend la séquence d'acides aminés DIHDKNAKFYPVTREGSYG pour utilisation dans le traitement du cancer du sein, où le cancer du sein, comprend une tumeur cancéreuse du sein triple négatif.

2. Anticorps pour utilisation selon la revendication 1, dans lequel l'anticorps comprend en outre un support acceptable du point physiologique ou un support acceptable du point de vue pharmaceutique.

3. Anticorps destiné à être utilisé selon la revendication 1, où l'anticorps :
i) entre en concurrence avec un anticorps comprenant les régions variables de chaîne lourde et de chaîne légère d'un diacorps de KS49, de KS41, de KS83 ou de KS89 ;
ii) partage 90% d'identité d'acides aminés avec des régions variables de chaîne lourde et de chaîne légère d'un diacorps de KS49, de KS41, de KS83 ou de KS89 ; et/ou
iii) comprend des séquences CDR identiques à celles d'un diacorps de KS49, de KS41, de KS83 ou de KS89.

4. Composition comprenant un anticorps selon l'une quelconque des revendications 1 à 3 et
i) au moins un agent anticancéreux supplémentaire ; et/ou
ii) un diagnostic compagnon,
pour utilisation dans le traitement du cancer du sein, où le cancer du sein comprend une tumeur cancéreuse du sein triple négatif.

5. Composition pour utilisation selon la revendication 4, où ledit au moins un agent anticancéreux supplémentaire :
i) est choisi dans le groupe constitué par des médicaments chimiothérapeutiques à base de platine, les taxanes, les inhibiteurs de tyrosine kinase, les anticorps anti-EGFR, les anticorps anti-ErbB2 et des combinaisons de ceux-ci ;
ii) comprend un inhibiteur de l'EGFR ;
iii) comprend un inhibiteur du VEGF.

6. Composition pour utilisation selon la revendication 5, où l'inhibiteur de l'EGFR :
i) Comprend un anticorps anti-EGFR ;
ii) est un inhibiteur à petite molécule de signalisation de l'EGFR.

7. Composition pour utilisation selon la revendication 6, où l'anticorps anti-EGFR :
i) comprend du cétuximab ;
ii) est choisi dans le groupe constitué par le matuzumab, le panitumumab et le nimotuzumab.

8. Composition pour utilisation selon la revendication 6, où l'inhibiteur à petite molécule de signalisation de l'EGFR est choisi dans le groupe constitué par le géfitinib, le lapatinib, le canertinib, le pélitinib, l'erlotinib HCL, PKI-166, PD158780 et AG 1478.

9. Composition pour utilisation selon la revendication 5, où l'inhibiteur du VEGF comprend un anticorps anti-VEGF comme le bevacizumab.

10. Composition pour utilisation selon la revendication 4, où le compagnon de diagnostic comprend un anticorps anti-EMP2.

11. Anticorps ou composition pour utilisation selon l'une quelconque des revendications 1 à 10, où l'anticorps est conjugué à un fragment effecteur.

12. Anticorps ou composition pour utilisation selon la revendication 11, où le fragment effecteur est un agent toxique comme la ricine.

13. Anticorps ou composition pour utilisation selon l'une quelconque des revendications 1 à 12, où le traitement comprend le blocage de l'invasivité du cancer.

14. Anticorps ou composition pour utilisation selon l'une quelconque des revendications 1 à 13, où les anticorps sont utilisés dans des thérapies de vaccination pour le cancer.

15. Anticorps ou composition pour utilisation selon l'une quelconque des revendications 1 à 14, où le traitement est un patient qui est un mammifère.

16. Anticorps ou composition pour utilisation selon l'une quelconque des revendications 1 à 15, où le traitement est un patient qui est un humain.
